(19) Europäisches Patentamt
European Patent Office
Office européen des brevets

(11) Veröffentlichungsnummer: **0 098 569**
**A2**

# (12) EUROPÄISCHE PATENTANMELDUNG

(21) Anmeldenummer: **83106513.1**

(22) Anmeldetag: **04.07.83**

(51) Int. Cl.[3]: **C 07 D 251/16**, C 07 D 251/18, C 07 D 239/42, C 07 D 239/46, C 07 D 405/04, C 07 D 409/04, C 07 D 411/04, C 07 D 405/06, C 07 D 411/06, C 07 D 409/06, A 01 N 47/36

(30) Priorität: **08.07.82 DE 3225471**
**23.12.82 DE 3247616**

(43) Veröffentlichungstag der Anmeldung: **18.01.84**
**Patentblatt 84/3**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **Humburg, Gerhard, Dr., Königsberger Weg 24, D-6238 Hofheim am Taunus (DE)**
Erfinder: **Willms, Lothar, Dr., Grüner Weg 4, D-5463 Unkel (DE)**
Erfinder: **Mildenberger, Hilmar, Dr., Fasanenstrasse 24, D-6233 Kelkheim (Taunus) (DE)**
Erfinder: **Bauer, Klaus, Dr., Kolpingstrasse 7, D-6054 Rodgau (DE)**
Erfinder: **Bieringer, Hermann, Dr., Eichenweg 26, D-6239 Eppstein/Taunus (DE)**
Erfinder: **Bürstell, Helmut, Dr., Am Hohlacker 65, D-6000 Frankfurt am Main 50 (DE)**

(54) **Neue heterocyclisch substituierte Sulfonylharnstoffe, Verfahren zu ihrer Herstellung und ihre Verwendung in der Landwirtschaft.**

(57) Verbindungen der Formel

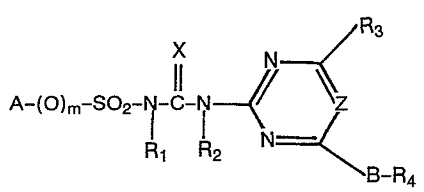

(I)

worin A einen (substituierten) aliphatischen, cycloaliphatischen oder Phenylrest, $R_1$ H oder Alkyl, $R_2$ H, Alkyl oder (substituiertes) Phenyl, X O oder S, $R_3$ H, Halogen, $NO_2$, CN, (Di)alkylamino, (subst.) Alkyl, (subst.) Alkoxy, (subst.) Alkylthio oder Alkoxycarbonyl, B eine einfache chemische Bindung oder eine (subst.) Methylen- oder Ethylengruppe, $R_4$ eine Carbonylgruppe oder eine davon abgeleitete funktionelle Gruppe, Z Stickstoff oder eine Methin-Gruppe und m 0 oder 1 bedeutet, sind wirksame Herbizide und Wachstumsregulatoren.

EP 0 098 569 A2

0098569

HOECHST AKTIENGESELLSCHAFT HOE 82/F 142 K Dr. AU/Rt

# Neue heterocyclisch substituierte Sulfonylharnstoffe, Verfahren zu ihrer Herstellung und ihre Verwendung in der Landwirtschaft

Es ist bereits bekannt, daß heterocyclisch substituierte Phenylsulfonylharnstoffe, wie z.B. N-(2-Chlorphenylsulfonyl)-N-(4-methoxy-6-methyl-1,3,5-triazin-2-yl)-harnstoff, herbizide oder pflanzenwuchsregulierende Eigenschaften aufweisen (vgl. z.B. NL-PS 121 788, DE-OS 27 15 786, EP 1 485, EP 1 514, EP 1 515, EP 4 163, EP 7 687, EP 9 419, EP 10 560, EP 23 140, EP 23 141, EP 23 422, EP 35 893).

Es wurde nun gefunden, daß heterocyclisch substituierte Phenyl-, Phenoxy-, (Halogen)Alkyl- und (Halogen)Alkoxysulfonylharnstoffe, die als heterocyclische Komponente einen mit einer Carbonylfunktion substituierten Pyrimidin- oder s-Triazinring enthalten bzw. die davon abgeleiteten funktionellen Derivate (z.B. Acetale, Halbacetale, Dioxolane, Mercaptale, Dithiolane, Aminale, Azomethine, Oxime, Oximether und Hydrazone) als Herbizide und Pflanzenwachstumsregulatoren geeignet sind.

Gegenstand der vorliegenden Erfindung sind demnach die neuen Verbindungen der Formel

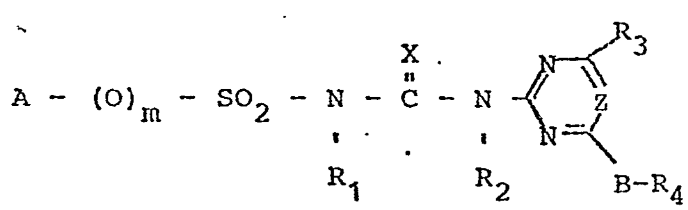

(I)

worin

A einen (ggf. durch bis zu 6 Halogenatomen substituierten) gesättigten oder ungesättigten aliphatischen Rest mit

bis zu 10 C-Atomen, der auch durch Sauerstoff unterbrochen sein kann; einen gesättigten cycloaliphatischen Rest mit 3 bis 12 C-Atomen oder einen ein- oder mehrfach ungesättigten Rest mit 5 - 12 C-Atomen, die gegebenenfalls durch bis zu 4 Halogenatome und/oder durch einen oder mehrere $(C_1-C_4)$-Alkyl- oder Halogenalkyl (letztere mit 1 - 3 Halogenatomen) bzw. durch einen $(C_1-C_4)$-Alkoxycarbonylrest substituiert sein können; einen bicyclischen gesättigten oder ein- oder zweifach ungesättigten aliphatischen Rest mit 7 bis 12 C-Atomen, welcher gegebenenfalls bis zu 6 Halogenatome oder eine oder mehrere $(C_1-C_4)$-Alkylrest tragen kann oder in dem eine $CH_2$-Brücke durch Sauerstoff ersetzt sein kann; oder einen Rest der Formel

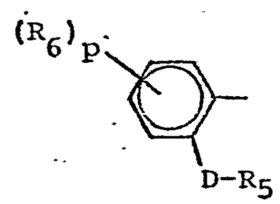

m null oder 1, bevorzugt 0,

X Sauerstoff oder Schwefel, bevorzugt Sauerstoff,

$R_1$ Wasserstoff oder $(C_1-C_4)$-Alkyl,

$R_2$ Wasserstoff, $(C_1-C_4)$-Alkyl oder Phenyl, das durch Halogen, $(C_1-C_4)$-Alkyl, $(C_1-C_4)$-Alkoxy, $NO_2$, $(C_1-C_4)$-Alkylthio und/oder Di-$(C_1-C_4)$-alkylamino substituiert sein kann, bevorzugt Wasserstoff oder $(C_1-C_4)$Alkyl,

$R_3$ Wasserstoff, Halogen, $NO_2$, CN, $(C_1-C_4)$-Alkylamino, Di-$(C_1-C_4)$-alkyl-amino, $(C_1-C_4)$-Alkyl, das ggf. durch Halogen, $(C_1-C_3)$-Alkoxy, $(C_1-C_3)$-Alkylthio, $(C_1-C_3)$-Alkylamino, Di-$(C_1-C_3)$-alkylamino oder $(C_1-C_4)$-Alkoxycarbonyl substituiert sein kann; $(C_1-C_4)$-Alkoxy oder $(C_1-C_4)$-Alkythio, die ggf. durch Halogen, $(C_1-C_4)$-Alkoxy oder $(C_1-C_4)$-Alkoxycarbonyl substituiert sein können, Allyloxy oder $(C_1-C_4)$-Alkoxycarbonyl;

B und D unabhängig voneinander jeweils eine einfache chemische Bindung oder eine Methylen- oder Ethylengruppe, die durch Halogen, $(C_1-C_4)$-Alkyl, $(C_1-C_4)$-Alkoxy, $(C_1-C_4)$-Alkylthio, $(C_1-C_4)$-Alkylamino oder Di-$(C_1-C_4)$-alkylamino substituiert sein kann;

$R_4$ ein Rest der Formel $-CO-R_7$;

$R_5$ Halogen, $NO_2$, $(C_1-C_4)$-Alkyl, $(C_1-C_4)$-Halogenalkyl oder $(C_1-C_4)$ Halogenalkoxy mit jeweils bis zu 5 Halogenatomen, $(C_1-C_4)$-Alkoxy, $(C_2-C_5)$-Alkenoxy, $/C_1-C_3)$-alkylthio, -Alkylsulfinyl oder -Alkylsulfonyl, $-SO_2N[(C_1-C_4)\text{-Alkyl}]_2$ oder $(C_1-C_4)$-Alkoxycarbonyl, einen Phenyl-bzw. Phenoxy-, Diphenyl-bzw. Diphenoxy-, Phenoxyphenyl-bzw. Phenoxyphenoxyrest, worin die Phenylreste durch Halogen, $NO_2$, $(C_1-C_4)$-Alkyl oder Alkoxy substituiert sein können, wobei die nichtaromatischen Reste bevorzugt sind,

$R_6$ gleich oder verschiedene Reste der Gruppe Halogen, $CF_3$, $NO_2$, $(C_1-C_4)$-Alkyl, $(C_1-C_4)$-Alkoxy oder $(C_2-C_5)$-Alkenoxy;

p 0 - 3;

$R_7$ Wasserstoff, $(C_1-C_4)$-Alkyl, $(C_1-C_4)$-Halogenalkyl, Phenyl, Halogenphenyl, $(C_1-C_4)$-Alkylphenyl oder $(C_1-C_4)$-Alkoxyphenyl, wobei die nichtaromatischen Reste bevorzugt sind,

Z Stickstoff oder eine Methin-Gruppe bedeuten,

sowie die funktionellen Derivate dieser Verbindungen und ihre physiologisch verträglichen Salze mit Basen und Säuren.

Unter "funktionellen Derivaten" sind Verbindungen mit Substituenten, die sich von der Carbonylgruppe $-CO-R_7$ ableiten, zu verstehen. Dazu gehören z.B. Acetale, Ketale, Halbacetale, Thioacetale (bzw. -ketale), Aminale, Halbaminale oder Verbindungen, in denen die Acetal-, Thioacetal- und/oder Aminalgruppen gemischt vorkommen oder ringförmig miteinander verknüpft sind, d.h. Verbindungen, in denen der Rest $R_4$ für eine Gruppe der Formel

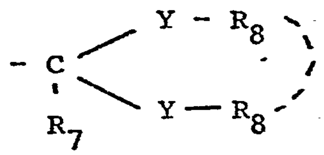

steht.

In dieser Formel steht Y für O, S und/oder $N-R_9$; beide $R_8$ sind $(C_1-C_4)$-Alkyl (einer davon kann auch Wasserstoff sein) oder bilden zusammen eine Ethylen oder Propylenbrücke und $R_9$ ist $(C_1-C_4)$-Alkyl oder Phenyl, das ggf. durch $(C_1-C_4)$-Alkyl, $(C_1-C_4)$-Alkoxy oder Halogen substituiert sein kann. Diese Derivate sind von besonderer Bedeutung.

Zu den funktionellen Derivaten gehören ferner Oxime, Oximether, Hydrazone, Semicarbazone oder Schiff-sche Basen (Azomethine) der Verbindungen I, d.h. Verbindungen, in denen $R_4$ für einen Rest der Formel

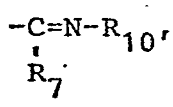, 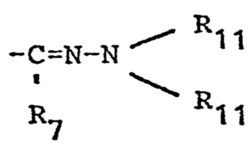 oder 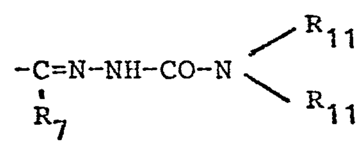

steht. In diesen bedeutet $R_{10}$ Hydroxy, $(C_1-C_4)$-Alkyl, $(C_1-C_4)$-Alkoxy, Phenyl oder Phenoxy (welch letztere durch Halogen, $(C_1-C_4)$-Alkyl oder $(C_1-C_4)$-Alkoxy substituiert sein können oder Benzyloxy und $R_{11}$ (die gleich oder verschieden sein können) Wasserstoff, $(C_1-C_4)$-Alkyl oder (ggf. durch Halogen, $(C_1-C_4)$-Alkyl oder $(C_1-C_4)$-Alkoxy substituiertes) Phenyl.

Unter "aliphatischen Resten" in A-Stellung sind Alkylreste, Alkenylreste mit einfacher oder konjugierter Doppelbindung oder Alkinylreste zu verstehen. "Halogen" bedeutet bevorzugt Fluor, Chlor oder Brom. Reste mit mindestens 3 C-Atomen zeichnen sich durch besonders

starke herbizide Wirkung aus.

Die neuenVerbindungen der allgemeinen Formel I lassen sich aus an sich bekannten bzw. nach bekannten Verfahren hergestellten Ausgangsmaterialien synthetisieren. Die Herstellungsverfahren sind dadurch gekennzeichnet, daß man

a) Verbindungen der Formel

$$A - (O)_m - SO_2 - N = C = O \qquad (II),$$

oder

$$A - (O)_m - SO_2 - \underset{\displaystyle R_1}{\underset{|}{N}} - \overset{\displaystyle X}{\overset{\|}{C}} - Y \qquad (III)$$

wobei für X = S Y = Cl und für X = O Y = Cl, $O(C_1-C_4)$-Alkyl oder $OC_6H_5$ bedeutet mit Verbindungen der Formel

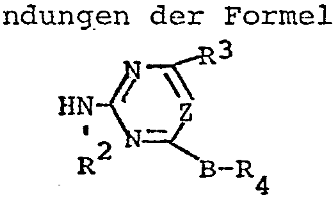

(IV)

oder

b) Verbindungen der Formel

$$A - (O)_m - SO_2 - \underset{\displaystyle R_1}{\underset{|}{NH}} \qquad (V)$$

mit Verbindungen der Formel

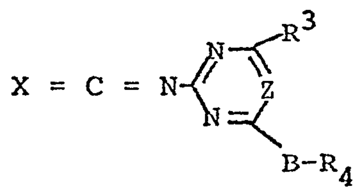

(VI)

oder

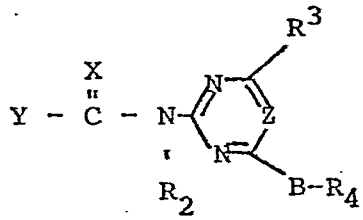

(VII)

umsetzt, wobei in Formel VII $R_2$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_1$-Position oder Salzbildung in andere Verbindungen der Formel I überführt, oder

c) Verbindungen der Formel

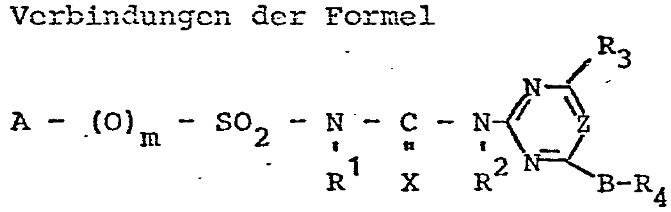

(I)

durch Umsetzung mit Alkoholen, Glycolen, Mercaptanen, Aminen, Hydroxylaminen, Anilinen, Hydrazinen oder Semicarbaziden in andere funktionelle Derivate der Formel I überführt.

Zu a) Die Umsetzung der Verbindungen II bzw. III und IV erfolgt vorzugsweise in inerten aprotischen Lösungsmitteln wie z.B. Acetonitril, Dichlormethan, Toluol, Tetrahydrofuran oder Dioxan bei Temperaturen zwischen 0°C und der Siedetemperatur des Lösungsmittels. Bei Anwendung von Ausgangsstoffen der Formel III arbeitet man gegebenenfalls in Gegenwart eines Säureakzeptors wie z.B. Kaliumcarbonat, Pyridin oder Triethylamin.

Zu b) Bei der Umsetzung der Verbindungen V mit VI bzw. VII arbeitet man gleichfalls vorzugsweise in den oben genannten inerten Lösungsmitteln gegebenenfalls unter Zusatz basischer Verbindungen, wie z.B. Kaliumcarbonat, Pyridin oder Triethylamin bei Temperaturen zwischen 0°C und der Siedetemperatur des Lösungsmittels.

Die nachträgliche Abspaltung von Halogenwasserstoff (HCl, HBr) aus aliphatischen halogenhaltigen Resten A erfolgt in bekannter Weise, z.B. mit Alkalialkoholat, alkoholischer Natron- oder Kalilauge, Triethylamin oder anderen säureabspaltenden Mitteln, gegebenenfalls in Gegenwart eines weiteren inerten Lösungs- oder Verdünnungsmittels (z.B. Toluol) bei Temperaturen zwischen Raum- und Siedetemperatur.

An vorhandene oder nachträglich gebildete Mehrfachbindungen in A-Position kann man Halogen ($Cl_2$, $Br_2$) oder Halogenwasserstoff in gleichfalls bekannter Weise anlagern und so gegebenenfalls zu neuen Verbindungen der Formel I gelangen. Die Bromierung bzw. Chlorierung wird in inerten organischen Lösungsmitteln wie z.B. Dichlormethan oder Chloroform unter Belichtung - beispielsweise mit ultraviolettem Licht - oder in Gegenwart von radikalisch zerfallenden Verbindungen - z.B. Azodiisobutyronitril - bei Temperaturen zwischen 0°C und der Siedetemperatur des Lösungsmittels durchgeführt. Die Anlagerung von Halogenwasserstoff gelingt in Gegenwart inerter Lösungsmittel (z.B. Toluol) mittels gasförmigem HCl oder HBr bei niedrigen Temperaturen, gegebenenfalls in Gegenwart eines Peroxidkatalysators.

Zur nachträglichen Alkylierung in $R_1$-Stellung arbeitet man vorzugsweise in inerten aprotischen Lösungsmitteln wie z.B. Dioxan oder Dimethylformamid unter Zusatz einer anorganischen Base, z.B. Natriumhydrid oder Kaliumcarbonat, bei

Temperaturen von 20°C bis zur Siedetemperatur des Lösungsmittels. Als Alkylierungsmittel werden z.B. Dimethylsulfat, Methyljodid oder Ethylbromid eingesetzt.

Zu c) Die Umwandlung von Verbindungen der Formel I mit z.B. einer Carbonylfunktion ($R_4$ = $-CO-R_7$) bzw. einer Ketalfunktion

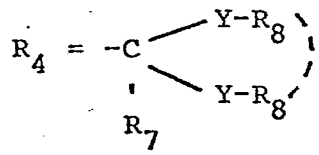

erfolgt nach im Prinzip bekannten Methoden, wie sie zusammenfassend in Houben-Weyl, Band VII (1), Seiten 413 - 488 bzw. Band VI (3), Seiten 204 - 270 beschrieben sind. Man verwendet für die Umsetzungen inerte protische oder aprotische Lösungsmittel, z.B. Ketone, wie Aceton oder Butanon, Alkohole wie Methanol, Ethanol oder Butanol, halogenierte Kohlenwasserstoffe wie Toluol, Ether wie Tetrahydrofuran oder Dioxan oder Acetonitril und arbeitet bei Temperaturen zwischen 0°C und der Siedetemperatur des Lösungsmittels. Im allgemeinen wird unter saurer bzw. basischer Katalyse gearbeitet. Als saure Katalysatoren verwendet man z.B. Salzsäure, Schwefelsäure, Toluolsulfonsäure oder Lewissäuren wie z.B. Bortrifluoridetherat; basische Katalysatoren sind Alkali- oder Erdalkalihydroxide, Oxide oder Carbonate, Salze organischer Säuren wie z.B. Natriumacetat und tertiäre Amine wie z.B. Triethylamin. Das bei den Umsetzungen entstehende Reaktionswasser wird entweder azeotrop oder mit Hilfe wasserbindender Mittel entfernt, z.B. mit physikalisch wirkenden Trockenmitteln wie Aluminiumoxid, Kieselgel oder Zeolithen.

Verbindungen der Formel I, in denen $R_1$ Wasserstoff bedeutet, können Salze bilden, bei denen H durch ein physiologisch verträgliches Kation ersetzt ist. Bei diesen Salzen handelt es sich vorzugsweise um Alkali-, Erdalkali-, Ammonium- oder organische Aminsalze.

Geeignete Basen zur Herstellung der erfindungsgemäßen Salze sind z.B. Kaliumcarbonat, Ammoniak oder Ethanolamin. Ebenso lassen sich Verbindungen, die in $R_3$- oder $R_4$-Stellung bzw. in Position B NH-Gruppen aufweisen, mit starken Säuren wie HCl, HBr oder $H_2SO_4$ in Salze überführen.

Die Ausgangsstoffe der Formel IV sind im Falle Z = Stickstoff neu, sie stellen infolge ihrer bifunktionellen Struktur interessante Zwischenprodukte dar, die außer für die Herstellung der erfindungsgemäßen Verbindungen der Formel I z.B. auch für die Herstellung von Pharmazeutika geeignet sind. Bisher gibt es kaum Beispiele für s-Triazine, die mit einer Formyl oder Ketofunktion bzw. mit deren funktionellen Derivaten substituiert sind. Mono-amino-s-triazine, die neben einem beliebigen Rest $R_3$ als weiteren Substituent eine Formyl- oder Ketofunktion bzw. deren funktionelle Derivate besitzen, werden im Rahmen der vorliegenden Anmeldung erstmals beschrieben.

Gegenstand der Erfindung sind daher auch die 2-Amino-s-triazine der Formel IV mit Z = Stickstoff, in der $R_2$, $R_3$ und B-$R_4$ die weiter oben angegebenen Bedeutungen haben.

Die Verbindungen der Formel IV mit Z = Stickstoff lassen sich nach Methoden herstellen, wie sie prinzipiell für die Synthese monofunktioneller Amino-s-triazine bekannt sind. Solche Methoden sind z.B. in J. org. Chem. 28, 1816 (1963); s-Triazines and Derivatives 217 (New York 1959); Chem. Pharm. Bull. 16, 474 (1968), Ber. 100, 1874 (1967) und Agric. Biol.

Chem. 35, 1572 (1971) beschrieben. Anstelle der dort verwendeten Ausgangsstoffe verwendet man entsprechende bifunktionelle Verbindungen der Formel X-B-$R_4$ (VIII) worin X für Reste der Formel CN, COOH, COCl oder COO($C_1$-$C_4$)-Alkyl steht. Beispiele hierfür sind Glyoxalsäureester, ß-Formylessigsäureester, Brenztraubsäureester, Acetessigester, oder davon abzuleitende funktionelle Derivate wie Dialkoxy-2-essigsäureester, Dialkoxyacetonitril, Dialkoyypropionsäureester oder 1.3-Dioxolan-2-carbonsäureester, 1.3-Dithiolan-2-carbonsäureester, 1.3-Dioxolan-2-essigsäureester oder 2-Methyl-1.3-dioxolan-2-essigsäureester. Es war nicht zu erwarten, daß diese bifunktionellen Verbindungen zu den erfindungsgemäßen Triazinen cyclisieren vielmehr mußte man mit einer Vielzahl von möglichen Reaktionsprodukten rechnen. Es ist deshalb überraschend, daß die bifunktionellen Verbindungen der Formel (VIII) in einfacher Weise und in guten Ausbeuten bei Anwendung der für monofunktionelle Amino-s-triazine bekannten Methoden zu den bifunktionellen Triazinen der Formel IV führen.

Die Herstellung der Verbindungen IV sei nachstehend am Beispiel des 2-Amino-4-diethoxy-methyl-6-methoxy-s-triazins veranschaulicht, das aus Diethoxyessigsäureethylester und Guanidino-O-methylisoharnstoff erhalten wird:

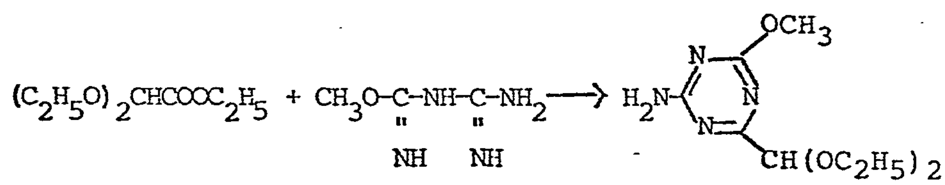

Die Umsetzungen erfolgen vorzugsweise in polaren Lösungsmitteln z.B. in Alkoholen wie Methanol oder Ethanol bzw. in aprotischen polaren Lösungsmitteln wie Acetonitril, DMF, Tetrahydrofuran und Dioxan bei Reaktionstemperaturen zwischen 0°C und der Siedetemperatur des Lösungsmittels. Da die als Reaktionspartner der Verbindungen VIII verwendeten Biguanidabkömmlinge wie N,N-Dimethylbiguanid oder Guanidino-O-methylisoharnstoff bzw. Amidinderivate wie z.B. O-Methyliso-harnstoff wegen mangelnder Beständigkeit in der Regel als Salze vorliegen, ist es notwendig sie zunächst in die freien Basen überzuführen.

Man verwendet dazu starke Basen, bzw. deren Lösungen wie Natriummethylat, Natriumethylat, Kalium-tert.-butylat bzw. deren alkoholische Lösungen; Natriumhydrid, Alkali- und Erdalkalihydroxide bzw. -carbonate oder tertiäre Amine wie Triethylamin, Ethyldiisopropylamin und Pyridin.

Die Ausgangsstoffe der Formel VIII sind bekannt oder können nach bekannten Methoden hergestellt werden [JACS 4580 (1954); JACS 2661 (1947); Beilstein 3 III 1139; 3 IV, 1494; 19 III/IIV, 3450 ff].

Die Amino-s-triazine der Formel IV (mit Z = Stickstoff) lassen sich in Abhängigkeit von den Resten $R_3$ und $B-R_4$ in andere Amino-s-triazine der Formel IV überführen. So erhält man durch Umsetzung von Verbindungen, in denen R = Cl ist, mit Alkoholen, Mercaptanen und Aminen unter Zusatz von Basen Verbindungen der Formel IV mit $R_3$ = O-Alkyl, S-Alkyl, NH-Alkyl oder $N-(Alkyl)_2$.

Bedeutet in den Verbindungen der Formel IV mit Z = Stickstoff der Rest $-B-R_4$ beispielsweise eine Carbonyl bzw. Ketal (Acetal)-funktion, so erhält man durch Umsetzung mit Alkoholen, Glycolen, Mercaptanen, Aminen, Hydroxylaminen, Anilinen, Hydrazinen oder Semicarbaziden andere neue S-Triazine der allgemeinen Formel IV. Diese Umwandlung erfolgt nach im Prinzip bekannten Methoden, wie sie zusammenfassend in Houben-Weyl, Band VII, (1), Seiten 413 - 488 bzw. Band VI (3), Seiten 204 - 270 beschrieben sind.

Man verwendet für diese - an sich bekannten - Folgereakreaktionen inerte protische oder aprotische Lösungsmittel, z.B. Ketone, Alkohole halogenierte Kohlenwasserstoffe, aromatische Kohlenwasserstoffe, Ether oder Acetonitril und arbeitet bei Temperaturen zwischen 0°C und der Siedetemperatur des Lösungsmittels. Im allgemeinen wird unter saurer bzw. basischer Katalyse gearbeitet. Das bei diesen Umsetzungen entstehende Reaktionswasser wird entweder azeotrop oder mit Hilfe wasserbindender Mittel entfernt.

Die Ausgangsstoffe der Formel IV mit Z = eine Methingruppe sind bekannt oder lassen sich nach im Prinzip bekannten Verfahren herstellen, z.B. durch Zyklisierung entsprechender Guanidinderivate mit entsprechend substituierten 1,3-Diketonen (vgl. z.B. "The Chemistry of Heterocyclic compounds", Vol. XVI (1962) und Supplement I (1970)). Verbindungen der Formel IV, in denen $R_4$ für den Rest $COR_7$ steht, können auch in der weiter oben beschriebenen Weise in andere Verbindungen der Formel IV überführt werden.

Die Sulfonylisocyanate der Formel II sind zum großen Teil bekannt oder lassen sich nach im Prinzip bekannten Verfahren in einfacher Weise herstellen (vgl. EP 15 683, DE-AS 1 211 165, DE-AS 1 226 565, DE-AS 1 230 016, DE-AS 1 568 640 sowie Chem. Ber. 105, 2791, 2800 (1972).

Die Sulfonylcarbamoyl- bzw. -thiocarbamoylchloride der Formel III lassen sich nach üblichen Methoden aus den Alkalisalzen der entsprechenden Sulfonamide der Formel V, welche aus der Literatur bekannt sind, durch Umsetzung mit Phosgen oder Thiophosgen darstellen.

Die für die Umsetzungen nach b) benötigten Iso(thio)cyanate der Formel VI sind neu und nach bekannten Verfahren zugänglich (vgl. Tetrahedron 29, 691 (1973); Japan. Kokai Sho-51-143686).

Gleiches gilt für die heterocyclischen Carbamoylchloride und Thiocarbamoylchloride der Formel VII (vgl. z.B. DE-AS 1 149 718 und DE-AS 2 238 870).

Sulfonylharnstoffe der Formel I, welche im aliphatischen Rest A eines oder mehrere asymmetrische Kohlenstoffatome enthalten, liegen in enantiomeren oder diastereomeren Formen vor. Im allgemeinen werden die entsprechenden erfindungsgemäßen Verbindungen als Racemate oder als Diastereomerengemische erhalten. Falls gewünscht, können die üblichen Techniken zur Trennung dieser Gemische in die sterisch einheitlichen Bestandteile angewendet werden. Auch durch Verwendung von sterisch einheitlichen Ausgangsmaterialien ist eine Reindarstellung der genannten Verbindungen möglich. Z.B. entstehen bei der Umsetzung von Aminoheterocyclen der Formel IV mit threo- bzw. erythro-1,2-Dihalogenalkyl-sulfonyl-isocyanaten diesen entsprechende threo- bzw. erythro-1,2-Dihalogenalkyl-sulfonyl-harnstoffe. Ferner können Sulfonylharnstoffe der Formel I, welche im aliphatischen Rest A eine oder mehrere Doppelbindungen enthalten, bei entsprechender olefinischer Substitution als E- oder als Z-Isomere vorkommen, deren Reindarstellung bzw. Trennung ebenfalls möglich ist. Werden z.B. ungesättigte Sulfonylisocyanate der allgemeinen Formel II als E- oder Z-Isomere eingesetzt, so werden die ungesättigten Sulfonylharnstoffe der Formel I in sterisch einheitlicher Form erhalten. Die Erfindung betrifft daher auch die einzelnen Enantiomeren und diastereomeren Formen der Verbindungen der Formel I.

Die Verbindungen der Formel I weisen eine ausgezeichnete herbizide Wirkung und in wichtigen Großkulturen eine sehr gute Selektivität auf. Sei eignen sich zu selektiven Bekämpfung zweikeimblättriger und grasartiger annueller und perennierender Unkräuter in landwirtschaftlich bedeutenden Kulturen wie z.B. Weizen, Gerste, Roggen, Reis, Mais, Zurckerrübe, Baumwolle und Soja im Vor- und Nachauflaufverfahren. Darüber hinaus besitzen sie in niedrigen Dosierungen hervorragende wachstumsregulatorische Eigenschaften bei Kulturpflanzen. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchsstauchung, eingesetzt werden. Des weiteren eignen

sie sich zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Werden die Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3 - 5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt.

Die vorliegenden Verbindungen eignen sich aus diesen Gründen hervorragend zur Bekämpfung von unerwünschtem Pflanzenwuchs in landwirtschaftlichen Nutzpflanzungen.

Gegenstand der Erfindung sind daher auch herbizide bzw. wachstumsregulierende Mittel, die gekennzeichnet sind durch

einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2 - 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungsmittel oder Inertstoff noch Netzmittel z.B. polyoxethylierte Alkylphenole, polyoxethylierte Fettalkohole, Alkyl- oder Alkylphenyl-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylarylsulfonsaure Kalziumsalze wie Ca-dodecylbenzosulfonat oder nichtionische Emulgatoren wie fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxyethylensorbitan-fettsäureester oderPolyoxethylen-sorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2 - 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z. B. bei Spritzpulvern und emulgierbaren Konzentraten mittels Wasser. Staubförmige und granulierte Zubereitungen sowie versprühbare Lösungen werden vor der Anwendung nicht mehr in weiteren inerten Stoffen verdünnt. Mit den äußeren Bedingungen wie Temperatur, Feuchtigkeit, u.s. variiert die erforderliche Aufwandmenge. Sie beträgt im allgemeinen

zwischen 0.005 und 10 kg/ha, vorzugsweise etwa 0.01 bis 5.0 kg/ha Wirkstoff.

Für einige Anwendungsgebiete kann es zweckmäßig sein, die neuen Herbizide mit einem oder mehreren Herbiziden gemeinsam anzuwenden, z.B. als "Tankmischung" oder in Form einer Fertigformulierung, um weitere vorteilhafte Wirkungen zu erzielen.

Die erfindungsgemäßen Wirkstoffe können mit anderen Herbiziden, Insektiziden und Fungiziden kombinert werden.

Zur Anwendung als Wachstumsregulatoren eignen sich Konzentrationen zwischen 0.01 und 1.25 kg/ha. Bevorzugt verwendet man wäßrige Dispersionen von Spritzpulvern oder Verdünnungen von emulgierbaren Konzentraten. Die Anwendung erfolgt im Nachauflauf. Bevorzugte Kulturen sind Mais und Tabak.

## HERSTELLUNGSBEISPIELE

### Beispiel 1

**N-(2-Chlorphenylsulfonyl)-N'-(4-diethoxymethyl-6-methyl-pyrimidin-2-yl)-harnstoff**

Zu 10.55 g (0.05 mol) 2-Amino-4-diethoxymethyl-6-methyl-pyrimidin in 50 ml Dichlormethan tropft man bei 0 bis 5 °C eine Lösung von 11 g (0.05 mol) 2-Chlorphenylsulfonylisocyanat in 20 ml Dichlormethan. Man rührt 18 h bei Raumtemperatur nach, setzt n-Hexan hinzu und saugt das ausgefallene Reaktionsprodukt ab. Man erhält 19.8 g (93 % d. Th.) an N-(2-Chlorphenylsulfonyl)-N'-(4-diethoxymethyl-6-methyl-pyrimidin-2-yl)-harnstoff vom Schmelzpunkt 131 °C.

### Beispiel 2

**N-(4-Diethoxymethyl-6-methyl-pyrimidin-2-yl)-N'-(2-methoxycarbonylphenylsulfonyl)-harnstoff**

Zu 10.55 g (0.05 mol) 2-Amino-4-diethoxymethyl-6-methyl-pyrimidin in 50 ml Dichlormethan tropft man bei 0 bis 5 °C eine Lösung von 12 g (0.05 mol) 2-Methoxycarbonylphenylsulfonylisocyanat in 20 ml Dichlormethan. Man rührt 12 Stunden bei Raumtemperatur nach, setzt n-Hexan hinzu und saugt das ausgefallene Reaktionsprodukt ab. Man erhält 19 g (84 % d. Th.) an N-(4-Diethoxymethyl-6-methyl-pyrimidin-2-yl)-N'-(2-methoxycarbonylphenylsulfonyl)-harnstoff vom Schmelzpunkt 170 °C.

Beispiel 3

N-(2-Chlorphenylsulfonyl)-N'-(4-formyl)-6-methyl-pyrimidin-2-yl)-harnstoff

34.3 g (0.08 mol) N-(2-Chlorphenylsulfonyl)-N'-(4-diethoxymethyl-6-methyl-pyrimidin-2-yl)-harnstoff (vergleiche Beispiel 1) werden in 350 ml Dioxan suspendiert und mit 20 ml halbkonzentrierter Salzsäure versetzt. Man rührt 18 Stunden bei Raumtemperatur nach, saugt das ausgefallene Produkt ab und wäscht den Niederschlag mit Dichlormethan. Man erhält 28 g (98 % d. Th.) an N-(2-Chlorphenylsulfonyl)-N'-(4-formyl-6-methyl-pyrimidin-2-yl)-harnstoff vom Schmelzpunkt 142 °C.

Beispiel 4

N-(4-Diethoxymethyl-6-methyl-pyrimidin-2-yl)-N'-(1-methyl-propen-1-yl-sulfonyl)-harnstoff

Zu 6.18 g (0.03 mol) 2-Amino-4-diethoxymethyl-6-methyl-pyrimidin in 50 ml Dichlormethan tropft man bei 0 bis 5 °C eine Lösung von 5.15 g (0.032 mol) 1-Methylpropen-1-yl-sulfonylisocyanat in 20 ml Dichlormethan. Man rührt 12 Stunden bei Raumtemperatur nach, setzt n-Hexan hinzu und saugt das ausgefallene Reaktionsprodukt ab. Man erhält 9,7 g (88 % d. Th.) an N-(4-Diethoxymethyl-6-methyl-pyrimidin-2-yl)-N'-(1-methyl-propen-1-yl-sulfonyl)-harnstoff vom Schmelzpunkt 100 - 102 °C.

Beispiel 5

N-(4-Dimethoxymethyl-6-methyl-pyrimidin-2-yl)-N'-(1-methyl-propen-1-yl-sulfonyl)-harnstoff

Zu 5.5g (0.03 mol) 2-Amino-4-dimethoxymethyl-6-methyl-pyrimidin in 50 ml Dichlormethan tropft man bei 0 bis 5 °C eine Lösung von 5.15 g (0.032 mol) 1- Methyl-propen-1-yl-sulfonylisocyanat in 20 ml Dichlormethan. Man rührt 12 Stunden bei Raumtemperatur nach, setzt n-Hexan hinzu und saugt das ausgefallene Produkt ab. Man erhält 9 g (87 % d. Th.) an N-(4-Dimethoxymethyl-6-methyl-pyrimidin-2-yl)-N'-(1-methyl-propen-1-yl-sulfonyl)-harnstoff vom Schmelzpunkt 115 - 117 °C.

In analoger Weise gemäß den in den Beispielen 1 bis 5 beschriebenen Verfahrensweisen erhält man unter Verwendung entsprechender Ausgangsstoffe die folgenden Verbindungen der Formel I von Tabelle I, wobei jeweils Z eine Methingruppe bedeutet:

0098569



Tabelle I

| Beispiel Nr. | A | m | $R_1$ | $R_2$ | $R_3$ | $B-R_4$ | Schmp. [°C] |
|---|---|---|---|---|---|---|---|
| 6 | 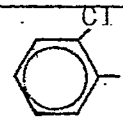 | 0 | H | H | $CH_3$ | $-CH(OCH_3)_2$ | 150 - 151 |
| 7 | 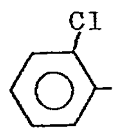 | 0 | H | H | $CH_3$ | -CH 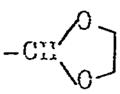 | 162 - 163 |
| 8 | 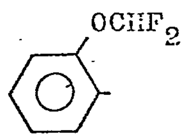 | 0 | H | H | $CH_3$ | $-CH(OC_2H_5)_2$ | 76 - 78 |
| 9 | 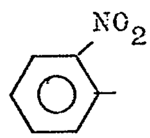 | 0 | H | H | $CH_3$ | $-CH(OC_2H_5)_2$ | 141 - 142 |
| 10 | 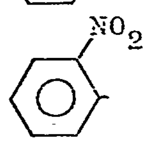 | 0 | H | H | $CH_3$ | $-CH(OCH_3)_2$ | 167-168 |
| 11 | 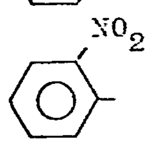 | 0 | H | H | $CH_3$ | -CH 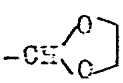 | |
| 12 | 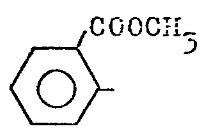 | 0 | H | H | $CH_3$ | $-CH(OCH_3)_2$ | 132 - 134 |
| 13 | 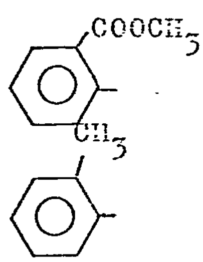 | 0 | H | H | $CH_3$ | -CH 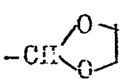 | 158 - 159 |
| 14 | | 0 | H | H | $CH_3$ | $-CH(OC_2H_5)_2$ | 126 - 127 |
| 15 | 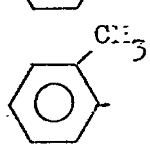 | 0 | H | H | $CH_3$ | $-CH(OCH_3)_2$ | |

| Bei-spiel Nr. | A | m | $R_1$ | $R_2$ | $R_3$ | $B-R_4$ | Schmp. [°C] |
|---|---|---|---|---|---|---|---|
| 16 | 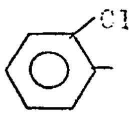 | 0 | H | H | $CH_3$ | 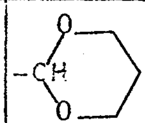 | |
| 17 | 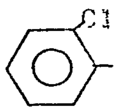 | 1 | H | H | $CH_3$ | $-CH(OC_2H_5)_2$ | Harz |
| 18 | 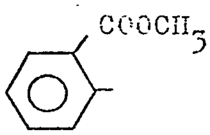 | 1 | H | H | $CH_3$ | $-CH(OC_2H_5)_2$ | 92 - 93 |
| 19 | 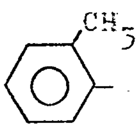 | 0 | H | H | $CH_3$ | -CHO | 140 -142 |
| 20 | 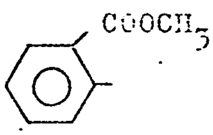 | 0 | H | H | $CH_3$ | -CHO | 158 -159 |
| 21 | 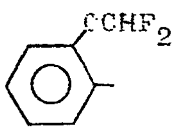 | 0 | H | H | $CH_3$ | -CHO | 172 - 173 |
| 22 | 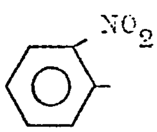 | 0 | H | H | $CH_3$ | -CHO | 145 - 148 |
| 23 | 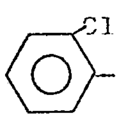 | 0 | H | H | Cl | $-CH(OC_2H_5)_2$ | 121 -122 |
| 24 | 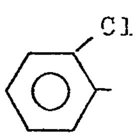 | 0 | H | H | $OCH_3$ | $-CH(OC_2H_5)_2$ | 106 - 107 |
| 25 | 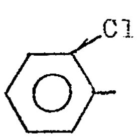 | 0 | H | H | $OC_2H_5$ | $-CH(OC_2H_5)_2$ | |

| Beispiel Nr. | A | m | $R_1$ | $R_2$ | $R_3$ | B - $R_4$ | F.P. [°C] |
|---|---|---|---|---|---|---|---|
| 26 | 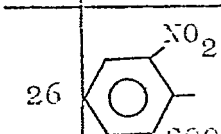 | 0 | H | H | Cl | $-CH(OC_2H_5)_2$ | 144 - 145 |
| 27 | 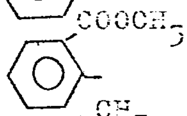 | 0 | H | H | Cl | $-CH(OC_2H_5)_2$ | |
| 28 | 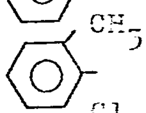 | 0 | H | H | Cl | $-\underset{CH_3}{C}H(OC_2H_5)_2$ | |
| 29 | 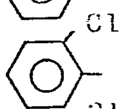 | 0 | H | H | $CH_3$ | $-C(OC_2H_5)_2$ | 167 - 168 |
| 30 | 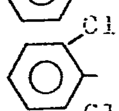 | 0 | H | H | $CH_3$ | $-CO-CH_3$ | 194 - 195 |
| 31 | 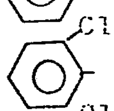 | 0 | H | H | $CH_3$ | $-CH=NOCH_3$ | 190 (Zers) |
| 32 | 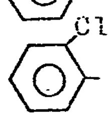 | 0 | H | H | $CH_3$ | $-CH=NOH$ | 195 (Zers) |
| 33 | 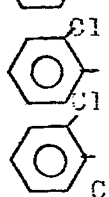 | 0 | H | H | $CH_3$ | -CH 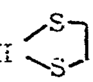 | 165 - 166 |
| 34 | | 0 | H | H | $CH_3$ | $-CH=NC_3H_7$ | |
| 35 | 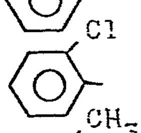 | 0 | H | H | $CH_3$ | $-CH[N(CH_3)_2]_2$ | |
| 36 | 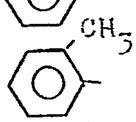 | 0 | H | H | $CH_3$ | $-CH=NOCH_3$ | |
| 37 | 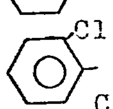 | 0 | H | H | $CH_3$ | $-CH=N-C_6H_5$ | |
| 38 | 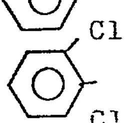 | 0 | H | H | $CH_3$ | $-CH=N-NHC_6H_5$ | |
| 39 | 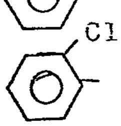 | 0 | H | H | $SC_2H_5$ | $-CH(OC_2H_5)_2$ | |

| Bei-spiel-Nr. | A | m | $R_1$ | $R_2$ | $R_3$ | B - $R_4$ | F.P. [°C] |
|---|---|---|---|---|---|---|---|
| 40 | 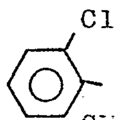 | 0 | H | H | $N(CH_3)_2$ | $-CH(OC_2H_5)_2$ | |
| 41 | 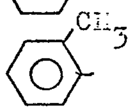 | 0 | H | H | $OCH_2$ $CH=CH_2$ | $-CH(OC_2H_5)_2$ | |
| 42 | 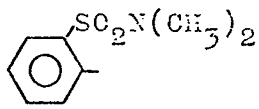 | 0 | H | H | $CH_3$ | $-CH(OCH_3)_2$ | 144-145 |
| 43 | 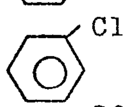 | 0 | H | H | $CH_3$ | $-CH(SC_2H_5)_2$ | 133 - 134 |
| 44 | 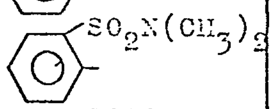 | 0 | H | H | Cl | $-CH(OC_2H_5)_2$ | |
| 45 | 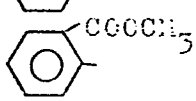 | 0 | H | H | $OC_2H_5$ | $-CH(OC_2H_5)_2$ | |
| 46 | $CCl_3CH_2-$ | 1 | H | H | $CH_3$ | $-CH(OC_2H_5)_2$ | |
| 47 | $CF_3CH_2-$ | 1 | H | H | $CH_3$ | $-CH(OCH_3)_2$ | |
| 48 | $(ClCH_2)_2\ CH-$ | 1 | H | H | Cl | $-CH(OC_2H_5)_2$ | |
| 49 | $CH_2=CH-CH_2-$ | 1 | H | H | Cl | $-CH(OCH_3)_2$ | |
| 50 | $ClCH_2CH_2-$ | 0 | H | H | Cl | $-CH(OC_2H_5)_2$ | |
| 51 | $ClCH_2CH_2-$ | 0 | H | H | Cl | -CH 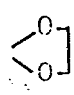 | |
| 52 | $BrCH_2CHBr-$ | 0 | H | H | $CH_3$ | $-CH=NOCH_3$ | |
| 53 | $CH_3CHClCH_2-$ | 0 | H | H | $CH_3$ | $-CH(OC_2H_5)_2$ | |

| Beispiel-Nr. | A | [illegible] | $R_1$ | $R_2$ | $R_3$ | B- $R_4$ | F.P. [°C] |
|---|---|---|---|---|---|---|---|
| 54 | $CH_3CHClCH_2$ | O | H | H | Cl | 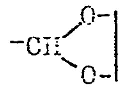 | |
| 55 | $CH_3CHClCH_2$ | O | H | H | $CH_3$ | -CHO | |
| 56 | $CH_3CH=CH-$ | O | H | H | $CH_3$ | $-CH(OCH_3)_2$ | |
| 57 | $CH_3CH=CH-$ | O | H | H | Cl | $-CH(OC_2H_5)_2$ | |
| 58 | $CH_3CH=CH-$ | O | H | H | $CH_3$ | $-CH[N(CH_3)_2]_2$ | |
| 59 | $CH_3CHCl-CHCl$ | O | H | H | $CH_3$ | -CHO | |
| 60 | $CH_3CHCl-CHCl$ | O | H | H | $CH_3$ | $-CH=N-C_6H_5$ | |
| 61 | $CH_3CHClCHCl$ | O | H | H | $OCH_3$ | $-CH(OC_2H_5)_2$ | |
| 62 | $CH_3CHClCHCl$ | O | H | H | $OCH_3$ | $-CH(OCH_3)_2$ | |
| 63 | $CH_3CH_2CH=CH$ | O | H | H | $CH_3$ | $-CH(OC_2H_5)_2$ | |

| Bei-spiel-Nr. | A | n | $R_1$ | $R_2$ | $R_3$ | B -$R_4$ | F.P. [°C] |
|---|---|---|---|---|---|---|---|
| 64 | $CH_3CH_2CH=CH-$ | 0 | H | H | $CH_3$ | $-CH(OCH_3)_2$ | |
| 65 | $CH_3-CH=C(CH_3)-$ | 0 | H | H | $CH_3$ | $-CHO$ | |
| 66 | $CH_3CH=C(CH_3)-$ | 0 | H | H | Cl | $-CH(OC_2H_5)_2$ | |
| 67 | $CH_3CH=C(CH_3)-$ | 0 | H | H | $CH_3$ | $-CH=NOCH_3$ | |
| 68 | $CH_3CH=C(CH_3)-$ | 0 | H | H | $OC_2H_5$ | $-CH(OC_2H_5)_2$ | |
| 69 | $CH_3CHClCCl(CH_3)-$ | 0 | H | H | $CH_3$ | $-CH(OCH_3)_2$ | |
| 70 | $CH_3CHBrCBr(CH_3)-$ | 0 | H | H | $CH_3$ | $-CH(OC_2H_5)_2$ | |
| 71 | $CH_3CCl=C(CH_3)-$ | 0 | H | H | Cl | $-CH(OCH_3)_2$ | |
| 72 | $CH_3CHCl-CH(CH_3)-$ | 0 | H | H | $CH_3$ | $-CH(OC_2H_5)_2$ | Harz |
| 73 | $CH_3CBr=C(CH_3)-$ | 0 | H | H | $CH_3$ | $-CH(OC_2H_5)_2$ | |

| Bei-spiel-Nr. | A | m | $R_1$ | $R_2$ | $R_3$ | B-$R_4$ | F.P. [°C] |
|---|---|---|---|---|---|---|---|
| 74 | $CH_3CHBrCHBr-$ | O | H | H | $CH_3$ | $-CH(OC_2H_5)_2$ | 43 - 46 |
| 75 | $CH_3CHBrCHBr-$ | O | H | H | Cl | $-CH(OC_2H_5)_2$ | |
| 76 | $CH_3CHBrCHBr-$ | O | H | H | $OCH_3$ | $-CH(OCH_3)_2$ | |
| 77 | $CH_3(CH_2)_4CH=CH-$ | O | H | H | Cl | $-CH(SC_2H_5)_2$ | |
| 78 | 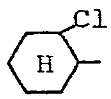 | O | H | H | $CH_3$ | $-CH(OC_2H_5)_2$ | |
| 79 | 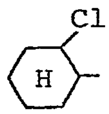 | O | H | H | $CH_3$ | $-CH(S\ C_2H_5)_2$ | |
| 80 | 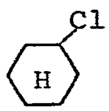 | O | H | H | $CH_3$ | $-\underset{CH_3}{C}(OC_2H_5)_2$ | |
| 81 | 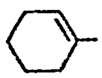 | O | H | H | $OCH_3$ | 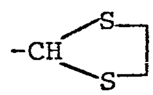 | |
| 82 | 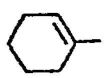 | O | H | H | $CH_3$ | 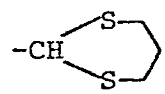 | |
| 83 | 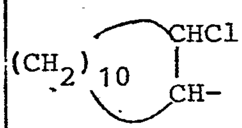 | O | H | H | $CH_3$ | $-CH(OCH_3)_2$ | |
| 84 | 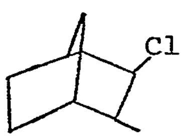 | O | H | H | $CH_3$ | $-CH(OC_2H_5)_2$ | |
| 85 | 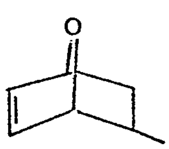 | O | H | H | $CH_3$ | $-\underset{CH_3}{C}(OCH_3)_2$ | |
| 86 | 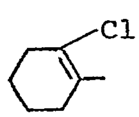 | O | H | H | $CH_3$ | $-CH(OC_2H_5)_2$ | |

| Beispiel-Nr. | | m | $R_1$ | $R_2$ | $R_3$ | B-$R_4$ | F.P. [°C] |
|---|---|---|---|---|---|---|---|
| 87 | 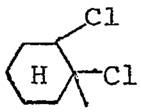 | O | H | H | $CH_3$ | $-CH(OCH_3)_2$ | |
| 88 | 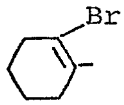 | O | H | H | $CH_3$ | $-CH(SC_2H_5)_2$ | |
| 89 | 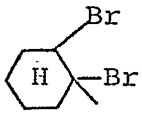 | O | H | H | $CH_3$ | $CH_3$<br>$-C(OC_2H_5)_2$ | |
| 90 | 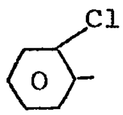 | O | H | H | $CH_3$ | 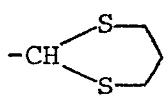 | 158 |
| 91 | 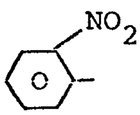 | O | H | H | $CH_3$ | $-C(OC_2H_5)_2$<br>$CH_3$ | 159-160 |
| 92 | 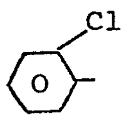 | O | H | H | $CH_3$ | $-C(OCH_3)_2$<br>$CH_3$ | 168-169 |
| 93 | 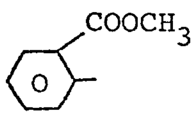 | O | H | H | $CH_3$ | $-C(OC_2H_5)_2$<br>$CH_3$ | 130-131 |
| 94 | 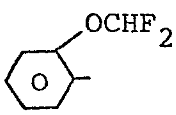 | O | H | H | $CH_3$ | $-C(OC_2H_5)_2$<br>$CH_3$ | 142-143 |
| 95 | 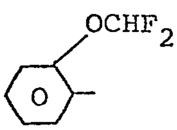 | O | H | H | $CH_3$ | $-CH(OCH_3)_2$ | 99-100 |
| 96 | 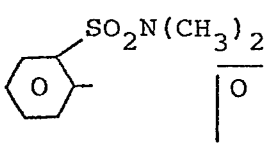 | O | H | H | $CH_3$ | $-CH(OC_2H_5)_2$ | 157-158 |

| Beispiel-Nr. | | m | $R_1$ | $R_2$ | $R_3$ | $B-R_4$ | F.P. [°C] |
|---|---|---|---|---|---|---|---|
| 97 | 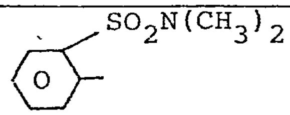 | O | H | H | $CH_3$ | 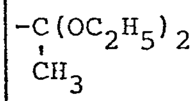 | 153-154 |
| 98 | 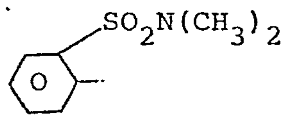 | O | H | H | $CH_3$ | $-CH(SC_2H_5)_2$ | |
| 99 | 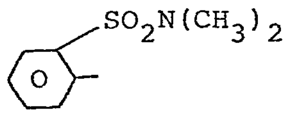 | O | H | H | $CH_3$ | 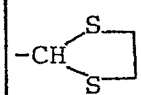 | 150-151 |
| 100 | 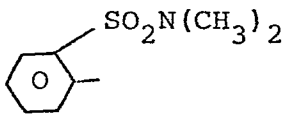 | O | H | H | $CH_3$ | 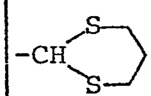 | 128-129 |
| 101 | 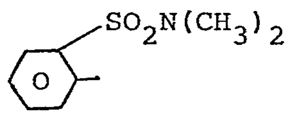 | O | H | H | $CH_3$ | 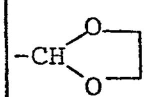 | 176-177 |
| 102 | 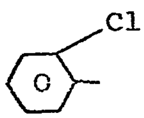 | O | H | H | $CH_3$ | 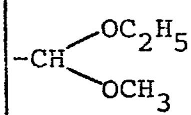 | |
| 103 | 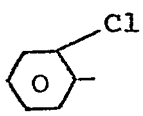 | O | H | H | $CH_3$ | 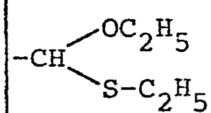 | |
| 104 | 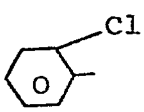 | O | H | H | $CH_3$ | 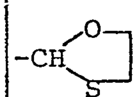 | 173-174 |
| 105 | 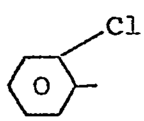 | O | H | H | $CH_3$ | 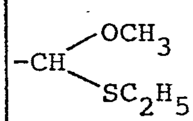 | |
| 106 | 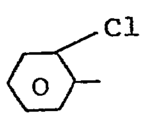 | O | H | H | $OCH_2CH_2OCH_3$ | $-CH(OCH_3)_2$ | |
| 107 | 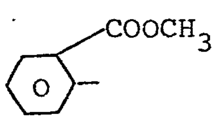 | O | H | H | $O(CH_2)_2OCH_3$ | $CH(OC_2H_5)_2$ | |
| 108 | 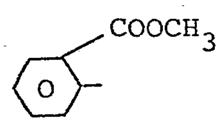 | O | H | H | $O(CH_2)_2OC_2H_5$ | $-CH(OCH_3)_2$ | |

| Bei-spiel-Nr. | | m | $R_1$ | $R_2$ | $R_3$ | $B-R_4$ | F.P. [°C] |
|---|---|---|---|---|---|---|---|
| 109 | 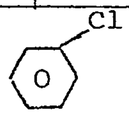 | O | H | H | $CH_3$ | 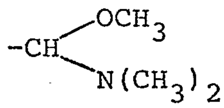 | |
| 110 | 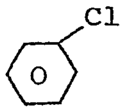 | O | H | H | $CH_3$ | $-CH=N-C_4H_9$ (n) | |
| 111 | 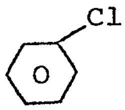 | O | H | H | $CH_3$ | $-C=N-NHC_6H_5$<br>$CH_3$ | |
| 112 | 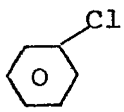 | O | H | H | $CH_3$ | $-CH=N-NHC_4H_9$ (n) | |
| 113 | 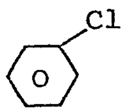 | O | H | H | $CH_3$ | $-C=N-NHC_4H_9$ (n)<br>$CH_3$ | |
| 114 | 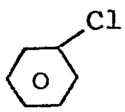 | O | H | H | $CH_3$ | $-CH=N-NH-C_6H_3(NO_2)_2$<br>(o,p) | |
| 115 | 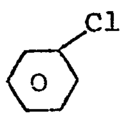 | O | H | H | $CH_3$ | $-CH=N-C_6H_4-Cl$ (p) | |
| 116 | 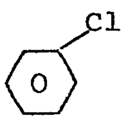 | O | H | H | $CH_3$ | 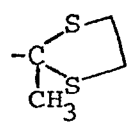 | |
| 117 | 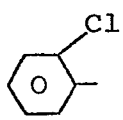 | O | H | H | $CH_3$ | 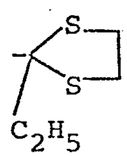 | |
| 118 | 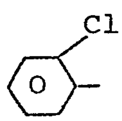 | O | H | H | $CH_3$ | 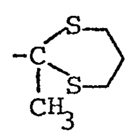 | |
| 119 | $CH_3-CH=C-$<br>$CH_3$ | O | H | H | $CH_3$ | 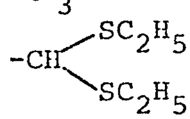 | |
| 120 | $CH_3-CH=C-$<br>$CH_3$ | O | H | H | $CH_3$ | 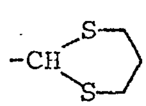 | |

| Bei-spiel-Nr. | | m | $R_1$ | $R_2$ | $R_3$ | $B-R_4$ | F.P. [°C] |
|---|---|---|---|---|---|---|---|
| 121 | $CH_3-CH=\underset{CH_3}{\underset{\mid}{C}}-$ | 0 | H | H | $CH_3$ | 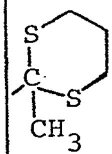 | |
| 122 | $CH_3-CHBr-CHBr-$ | 0 | H | H | $CH_3$ | 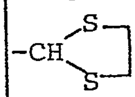 | |
| 123 | $CH_3-CHBr-CHBr-$ | 0 | H | H | $OCH_3$ | $-CH(SC_2H_5)_2$ | |
| 124 | $CH_3-CHCl-CHCl-$ | 0 | H | H | $OC_2H_5$ | 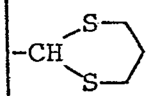 | |
| 125 | $CH_3-CH=\underset{CH_3}{\underset{\mid}{C}}-$ | 0 | H | H | $O(CH_2)_2OCH_3$ | $-CH(OC_2H_5)_2$ | |
| 126 | $CH_3-CH=\underset{CH_3}{\underset{\mid}{C}}-$ | 0 | H | H | $O(CH_2)_2OC_2H_5$ | $-CH(OCH_3)_2$ | |
| 127 | $CH_3CHBr-CHBr$ | 0 | H | H | $O(CH_2)_2OCH_3$ | 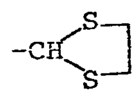 | |
| 128 | $CH_3CHClCHCl-$ | 0 | H | H | $-S-CH_3$ | $-CH(SC_3H_7)_2$ | |
| 129 | 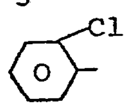 | 0 | H | H | $CH_3$ | $-\underset{CH_2Cl}{\underset{\mid}{C}}(OC_2H_5)_2$ | |
| 130 | 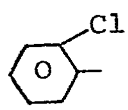 | 0 | H | H | $CH_3$ | $-\underset{CH_2CH_2Cl}{\underset{\mid}{C}}(OC_2H_5)_2$ | |
| 131 | 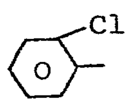 | 0 | H | H | $CH_3$ | $-\overset{O}{\overset{\|}{C}}-CH_2-CHCl_2$ | |
| 132 | 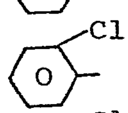 | 0 | H | H | $CH_3$ | $-CO-C_6H_4Cl(p)$ | |
| 133 | 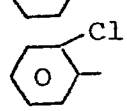 | 0 | H | H | $CH_3$ | $-\overset{OC_2H_5}{\overset{\mid}{\underset{\underset{OC_2H_5}{\mid}}{C}}}-C_6H_4CH_3(p)$ | |
| 134 | 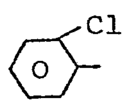 | 0 | H | H | $CH_3$ | $CH[N(C_2H_5)_2]_2$ | |

| Bei-spiel-Nr. | | m | $R_1$ | $R_2$ | $R_3$ | $B-R_4$ | F.P. [°C] |
|---|---|---|---|---|---|---|---|
| 135 | 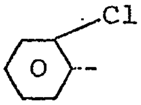 | O | H | H | $CH_3$ | $-C(CH_3)[N(C_2H_5)_2]_2$ | |
| 136 | 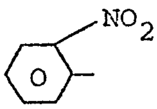 | O | H | H | $CH_3$ | $-CH=NOCH_3$ | |
| 137 | 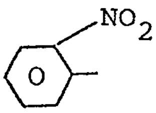 | O | H | H | $CH_3$ | $-CH=NOCH_2C_6H_5$ | |
| 138 | 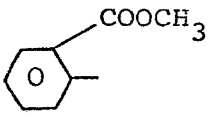 | O | H | H | $CH_3$ | $-C(CH_3)=NOCH_3$ | |
| 139 | 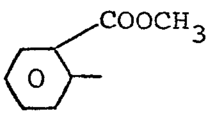 | O | H | H | H | $-C(=O)-CH_3$ | |
| 140 | 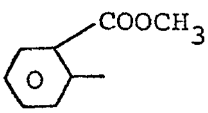 | O | H | H | H | $-CH(OC_2H_5)_2$ | |
| 141 | 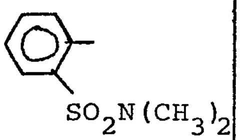 | O | H | H | $CH_3$ | $-CCH_3(OCH_3)_2$ | 153 |
| 142 | 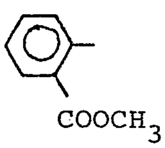 | O | H | H | $CH_3$ | $-CCH_3(OCH_3)_2$ | 146 - 147 |
| 143 | 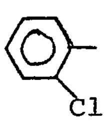 | O | H | H | $CH_2-O$ $H_3C$ | $-CH(OC_2H_5)_2$ | 116-117 |
| 144 | 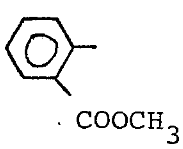 | O | H | H | $CH_3$ | 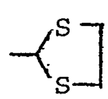 | 133-134 |

| Bei-spiel-Nr. | A | m | $R_1$ | $R_2$ | $R_3$ | $-B-R^4$ | Schmp. [°C] |
|---|---|---|---|---|---|---|---|
| 145 | 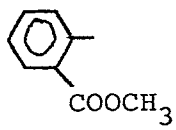 | O | H | H | $CH_3$ | 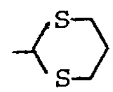 | 158 |
| 146 | 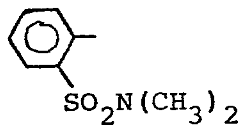 | O | H | H | $CH_3$ | 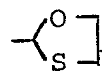 | 120-122 |
| 147 | 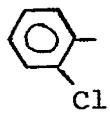 | O | H | H | $CH_3$ | $-CH(SCH_3)_2$ | 148-149 |

Beispiel 148

N-(2-Chlorphenylsulfonyl)-N'-(4-Diethoxymethyl-6-methoxy-s-triazin-2-yl)-harnstoff

Zu 11.4 g (0.05 mol) 2-Amino-4-diethoxymethyl-6-methoxy-s-triazin in 50 ml Dichlormethan tropft man bei 0 bis 5°C eine Lösung von 11 g (0.05 mol) 2-Chlorphenylsulfonylisocyanat in 20 ml Dichlormethan. Man rührt 18 h bei Raumtemperatur nach, setzt n-Hexan hinzu und saugt das ausgefallene Reaktionsprodukt ab. Man erhält 18.3 g (82 % d. Th.) an N-(2-Chlor-phenylsulfonyl)-N'-(4-diethoxymethyl-6-methoxy-s-triazin-2-yl)-harnstoff vom Schmelzpunkt 126 - 127°C.

In analoger Weise erhält man unter Verwendung entsprechender Ausgangsstoffe die in nachfolgender Tabelle angegebenen Verbindungen der Formel I, wobei jeweils Z = Stickstoff bedeutet.

| Bsp.-Nr. | A | m | $R_1$ | $R_2$ | $R_3$ | $-B-R_4$ | Schmp. [°C] |
|---|---|---|---|---|---|---|---|
| 149 | 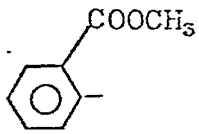 | 0 | H | H | $OCH_3$ | 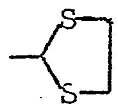 | 129 - 131 |
| 150 | 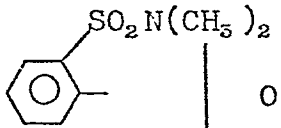 | 0 | H | H | $OCH_3$ | $-CH(OC_2H_5)_2$ | 132 - 133 |
| 151 | 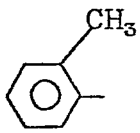 | 0 | H | H | $OCH_3$ | $-CHO$ | |
| 152 | 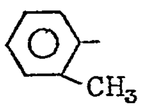 | 0 | H | H | $OCH_3$ | 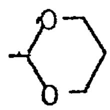 | |
| 153 | 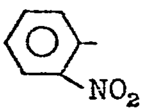 | 0 | H | H | Cl | $-CH(OC_2H_5)_2$ | |
| 154 | 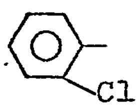 | 0 | H | H | $OCH_3$ | $-CH(OCH_3)_2$ | 137 - 138 |
| 155 | 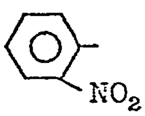 | 0 | H | H | $OCH_3$ | $-CH(OC_2H_5)_2$ | 141 - 142 |
| 156 | 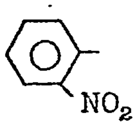 | 0 | H | H | $OCH_3$ | $-CH(OCH_3)_2$ | 154 - 155 |
| 157 | 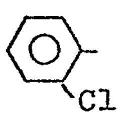 | 0 | H | H | $OCH_3$ | $-CHO$ | |

| Bsp.-Nr. | A | m | $R_1$ | $P_2$ | $R_3$ | $-B-R_4$ | Schmp. [°C] |
|---|---|---|---|---|---|---|---|
| 158 | 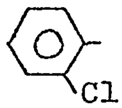 | 0 | H | H | $OCH_3$ | $-CO-CH_3$ | |
| 159 | 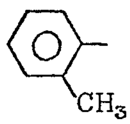 | 0 | H | H | $OC_2H_5$ | $-CH=NOH$ | |
| 160 | 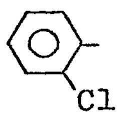 | 0 | H | H | $SC_2H_5$ | $-CH(OC_2H_5)_2$ | |
| 161 | 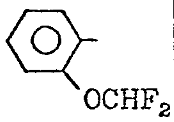 | 0 | H | H | $OCH_3$ | $-CH(OC_2H_5)_2$ | 95 - 96 |
| 162 | 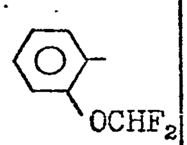 | 0 | H | H | $OCH_3$ | $-CH(OCH_3)_2$ | 103 - 104 |
| 163 | 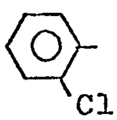 | 0 | H | H | $OCH_3$ | $-CH=NOCH_3$ | |
| 164 | 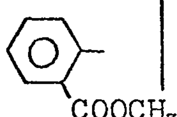 | 0 | H | H | $OCH_3$ | $-CH(OCH_3)_2$ | 119 -121 |
| 165 | 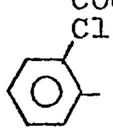 | 0 | H | H | $OCH_3$ | 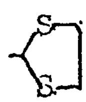 | 154 - 155 |
| 166 | 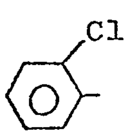 | 0 | H | H | $OCH_3$ | $-C(OC_2H_5)_2$<br>$CH_3$ | 148 - 149 |
| 167 | 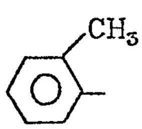 | 0 | H | H | $OC_2H_5$ | $-CH=N-C_6H_5$ | |

| Bsp.-Nr. | A | m | $R_1$ | $R_2$ | $R_3$ | $-B-R_4$ | Schmp. [°C] |
|---|---|---|---|---|---|---|---|
| 168 | 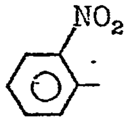 | 0 | H | H | $OCH_3$ | $-CH=N-NHC_6H_5$ | |
| 169 | 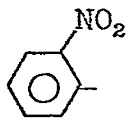 | 0 | H | H | $OC_3H_7$ | $-CH(OC_2H_5)_2$ | |
| 170 | 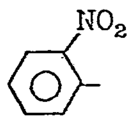 | 0 | H | H | $OCH_2-CH=CH_2$ | $-CH(SC_2H_5)_2$ | |
| 171 | 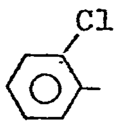 | 0 | H | H | $OCH_3$ | 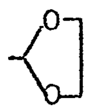 | 156-157 |
| 172 | 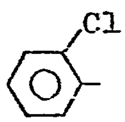 | 0 | H | H | $OCH_3$ | 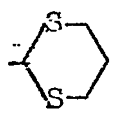 | 147-149 |
| 173 | 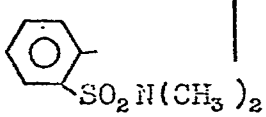 | 0 | H | H | $OCH_3$ | 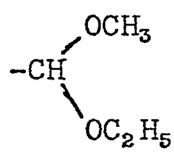 | |
| 174 | 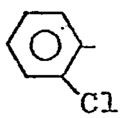 | 0 | H | H | $OCH_3$ | $-CH[N(C_2H_5)_2]_2$ | |
| 175 | 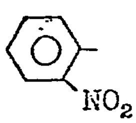 | 0 | H | H | $OCH_3$ | 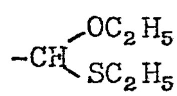 | |
| 176 | 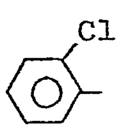 | 0 | H | H | $OCH_3$ | 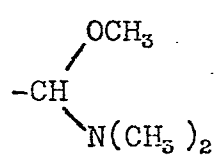 | |

| Bsp.-Nr. | A | m | $R_1$ | $R_2$ | $R_3$ | $-B-R_4$ | Schmp. /°C/ |
|---|---|---|---|---|---|---|---|
| 177 | 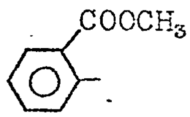 | 0 | H | H | $OCH_3$ | $-CH(OC_2H_5)_2$ | 84 -85 |
| 178 | 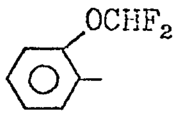 | 0 | H | H | $OCH_3$ | 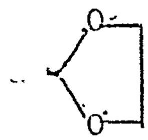 | 87 -88 |
| 179 | 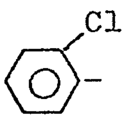 | 0 | H | H | $OCH_3$ | $-CH=N-C_4H_9$ (n) | |
| 180 | 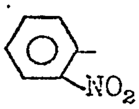 | 0 | H | H | $OC_2H_5$ | $-CH=N-NHC_4H_9$ (n) | |
| 181 | 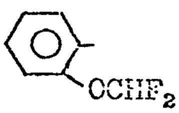 | 0 | H | H | $OCH_3$ | 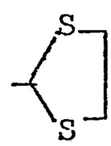 | 120-121 |
| 182 | 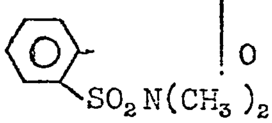 | 0 | H | H | $OCH_3$ | 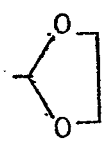 | 159-160 |
| 183 | 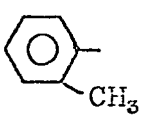 | 0 | H | H | $OCH_3$ | $-C(CH_3)=N-NHC_4H_9$ (n) | |
| 184 | 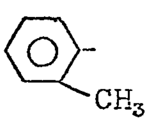 | 0 | H | H | $OCH_3$ | $-CH=N-C_6H_4-Cl$(p) | |
| 185 | 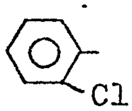 | 0 | H | H | $OCH_3$ | $-CH=N-NH-C_6H_3(NO_2)_2$ (o,p) | |

| Bsp.-Nr. | A | m | $R_1$ | $R_2$ | $R_3$ | -B-$R_4$ | Schmp. [°C] |
|---|---|---|---|---|---|---|---|
| 186 | 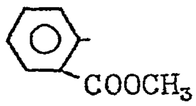 | 0 | H | H | $OCH_3$ | 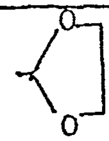 | 131 - 132 |
| 187 | 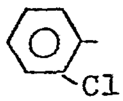 | 0 | H | H | $OC_2H_5$ | $-CH(OC_2H_5)_2$ | 132 - 133 |
| 188 | 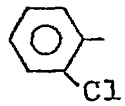 | 0 | H | H | $N(CH_3)_2$ | $-CH(OC_2H_5)_2$ | 151 - 152 |
| 189 | 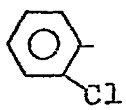 | 0 | H | H | $OCH_3$ | 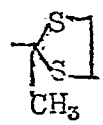 | |
| 190 | 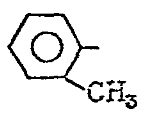 | 0 | H | H | $OCH_3$ | 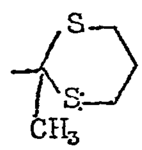 | |
| 191 | 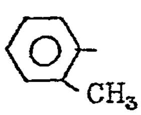 | 0 | H | H | $OCH_3$ | 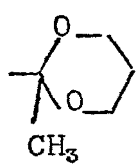 | |
| 192 | 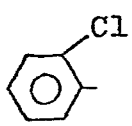 | 0 | H | H | $OCH_3$ | 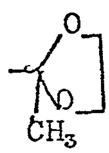 | 137 - 139 |
| 193 | 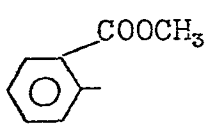 | 0 | H | H | $OC_2H_5$ | $-CH(OC_2H_5)_2$ | 129 - 130 |
| 194 | 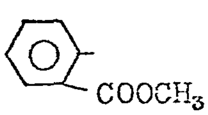 | 0 | H | H | $N(CH_3)_2$ | $-CH(OC_2H_5)_2$ | 99 |

| Bsp. Nr. | A | m | $R_1$ | $R_2$ | $R_3$ | $-B-R_4$ | Schmp. [°C] |
|---|---|---|---|---|---|---|---|
| 195 | 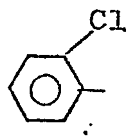 | 0 | H | H | $N(CH_3)_2$ | $-CH(OCH_3)_2$ | 162 - 163 |
| 196 | 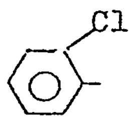 | 0 | H | H | $OCH_3$ | $-CO-CH_2-CHCl_2$ | |
| 197 | 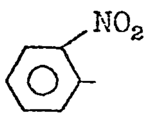 | 0 | H | H | $OCH_3$ | $-CH=NOCH_2C_6H_5$ | |
| 198 | 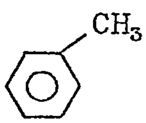 | 0 | H | H | $OCH_3$ | $-\underset{CH_3}{\underset{\vert}{C}}= NOCH_3$ | |
| 199 | 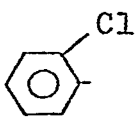 | 0 | H | H | $OCH_3$ | 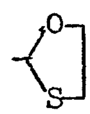 | 152 - 153 |
| 200 | 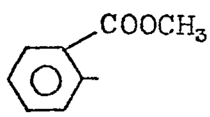 | 0 | H | H | $OCH_3$ | 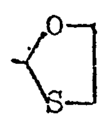 | 133 - 135 |
| 201 | 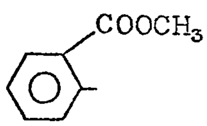 | 0 | H | H | $OCH_3$ | 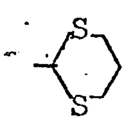 | 135 - 136 |
| 202 | 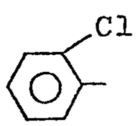 | 0 | H | H | $OCH_3$ | $-CH_2-CH(OC_2H_5)_2$ | |
| 203 | 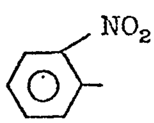 | 0 | H | H | $OCH_3$ | $-CH_2$ 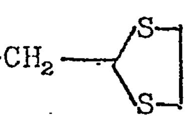 | |
| 204 | 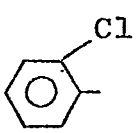 | 0 | H | H | $OCH_3$ | $-CH(SCH_3)_2$ | 129 - 130 |

| Bsp.-Nr. | A | m | $R_1$ | $R_2$ | $R_3$ | $-B-R_4$ | Schmp. [°C] |
|---|---|---|---|---|---|---|---|
| 205 | 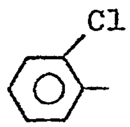 | O | H | H | $N(CH_3)_2$ | 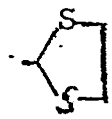 | 148 - 150 |
| 206 | 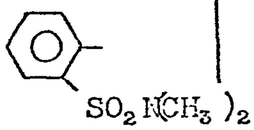 | O | H | H | $OCH_3$ | 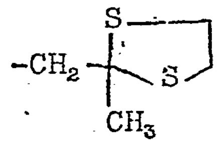 | |
| 207 | 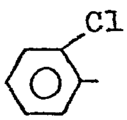 | O | H | H | $N(CH_3)_2$ | $-CH_2-CH_2-CH(OCH_3)_2$ | |
| 208 | 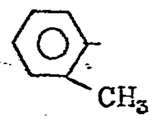 | O | H | H | $OC_2H_5$ | 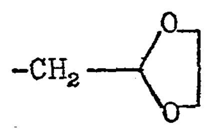 | |
| 209 | 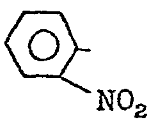 | O | H | H | $OCH_3$ | 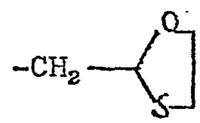 | |
| 210 | 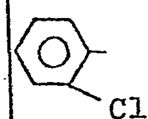 | O | H | H | $OCH_3$ | 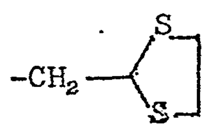 | |
| 211 | 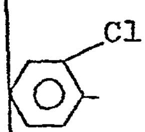 | O | H | H | $O(CH_2)_2OC_2H_5$ | $-CH(OCH_3)_2$ | Harz |
| 212 | 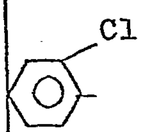 | O | H | H | $O(CH_2)_2OC_2H_5$ | $-CH(OC_2H_5)_2$ | Harz |

| Bsp. Nr. | A | m | $R_1$ | $R_2$ | $R_3$ | $-B-R_4$ | Schmp. [°C] |
|---|---|---|---|---|---|---|---|
| 213 | 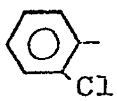 | 0 | H | H | $OCH_3$ | $-CH_2-$ 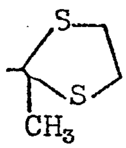 | 137-138 |
| 214 | 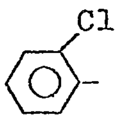 | 0 | H | H | $OCH_3$ | $-CH_2-$ 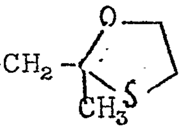 | 117-118 |
| 215 | 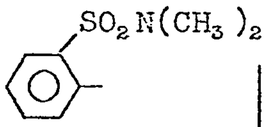 | 0 | H | H | $OCH_3$ | $-CH(OCH_3)_2$ | Harz |
| 216 | 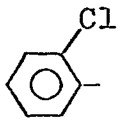 | 0 | H | H | $N(CH_3)_2$ | $-CH_2-CH_2-CH(OC_2H_5)_2$ | |
| 217 | 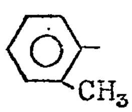 | 0 | H | H | $N(CH_3)_2$ | $-CH_2CH_2-$ 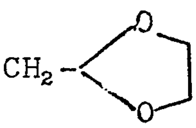 | |
| 218 | 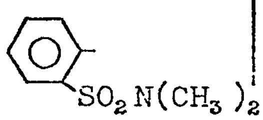 | 0 | H | H | $OCH_3$ | 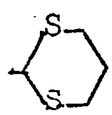 | 155-156 |
| 219 | 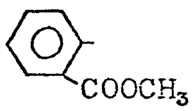 | 0 | H | H | $OC_2H_5$ | $-CH(OCH_3)_2$ | 148-149 |
| 220 | 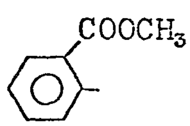 | 0 | H | H | $N(CH_3)_2$ | $-CH(OCH_3)_2$ | 150-151 |
| 221 | 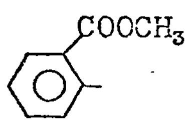 | 0 | H | H | $OCH_3$ | $-CH(SCH_3)_2$ | 148-149 |
| 222 | 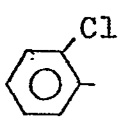 | 0 | H | H | $OCH_3$ | $-CH_2-CH_2-CH(OCH_3)_2$ | |
| 223 | 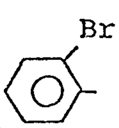 | 0 | H | H | $OCH_3$ | $-CH_2-CH_2-CH(OC_2H_5)_2$ | |

| Bsp. Nr. | A | m | $R_1$ | $R_2$ | $R_3$ | $-B-R_4$ | Schmp. [°C] |
|---|---|---|---|---|---|---|---|
| 224 | 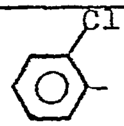 | 0 | H | H | $OCH_3$ | $-CH_2.CH_2$ 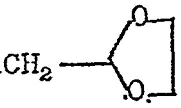 | |
| 225 | 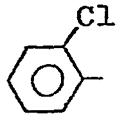 | 0 | H | H | $OCH_3$ | $-CH_2-CH(OCH_3)_2$ | 154 - 155 |
| 226 | 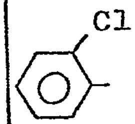 | 0 | H | H | $OCH_3$ | $-CH_2$ 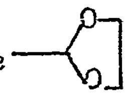 | |
| 227 | 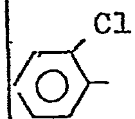 | 0 | H | H | $OCH_3$ | $-CH_2$ 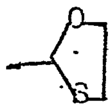 | |
| 228 | 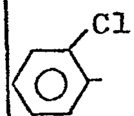 | 0 | H | H | $N(CH_3)_2$ | $-CH(SC_2H_5)_2$ | 126 - 127 |
| 229 | 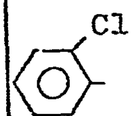 | 0 | H | H | $N(CH_3)_2$ | $-CH(SCH_3)_2$ | 165 - 166 |
| 230 | 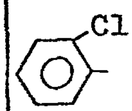 | 0 | H | H | $N(CH_3)_2$ | 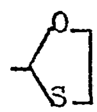 | |
| 231 | 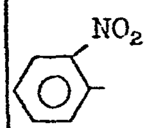 | 0 | H | H | $OCH_3$ | $-CH_2$ 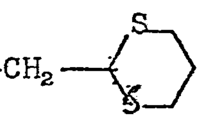 | |
| 232 | 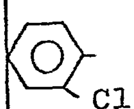 | 0 | H | H | $OCH_3$ | $-CH_2$ 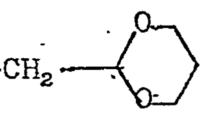 | |
| 233 | 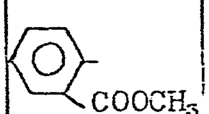 | 0 | H | H | $OCH_3$ | $-CH_2$ 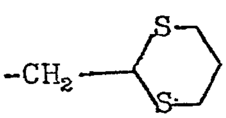 | |
| 234 | 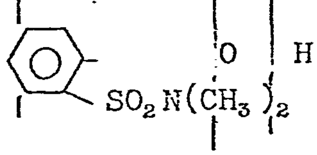 | 0 | H | H | $OCH_3$ | $-CH_2$ 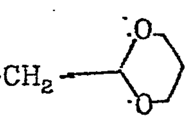 | |

| Bsp. Nr. | A | m | $R_1$ | $R_2$ | $R_3$ | $-B-R_4$ | Schmp. [°C] |
|---|---|---|---|---|---|---|---|
| 235 | 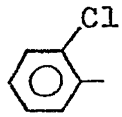 | O | H | H | $OCH_3$ | $-CH_2-CH2-$ 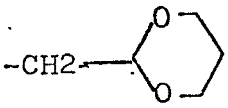 | |
| 236 | 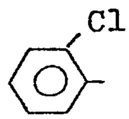 | O | H | H | $OCH_3$ | $-CH_2-CH_2-$ 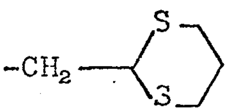 | |
| 237 | 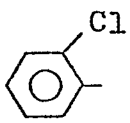 | O | H | H | $OC_2H_5$ | $-CH(OCH_3)_2$ | Harz |
| 238 | 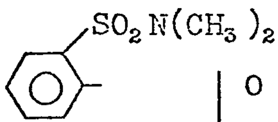 | O | H | H | $OCH_3$ | 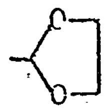 | 98 - 99 |
| 239 | 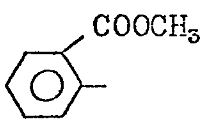 | O | H | H | $O(CH_2)_2OC_2H_5$ | $-CH(OC_2H_5)_2$ | Harz |
| 240 | 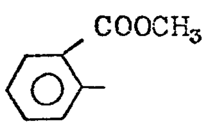 | O | H | H | $O(CH_2)_2OC_2H_5$ | $-CH(OCH_3)_2$ | Harz |
| 241 | 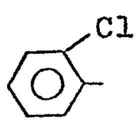 | O | H | H | $OCH_3$ | $-CH_2-$ 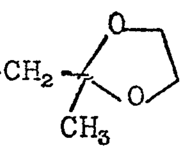 | 121-124 |
| 242 | 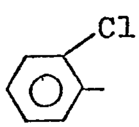 | O | H | H | $OCH_3$ | $-CH_2-$ 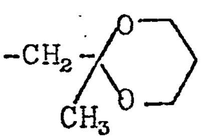 | |
| 243 | 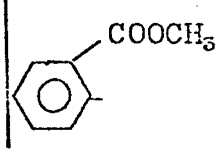 | O | H | H | $OCH_3$ | 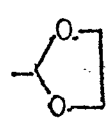 | 117-119 |

| Bsp.- Nr. | A | m | $R_1$ | $R_2$ | $R_3$ | $-B-R_4$ | Schmp. [°C] |
|---|---|---|---|---|---|---|---|
| 244 | 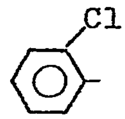 | 0 | H | H | $OCH_3$ | 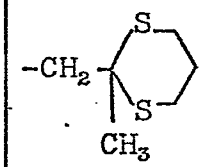 | |
| 245 | 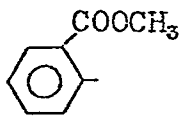 | 0 | H | H | $OCH_3$ | 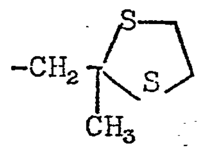 | 132 - 133 |
| 246 | 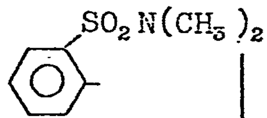 | 0 | H | H | $OCH_3$ | 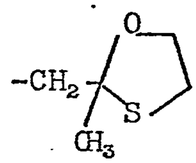 | 150 - 151 |
| 247 | 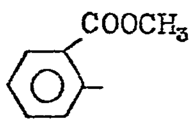 | 0 | H | H | $OCH_3$ | 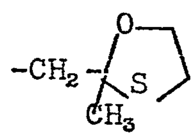 | |
| 248 | 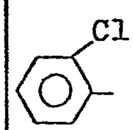 | 0 | H | H | $OCH_3$ | $-CH_2-COCH_3$ | |
| 249 | 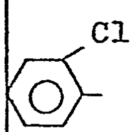 | 0 | H | H | $OCH_3$ | 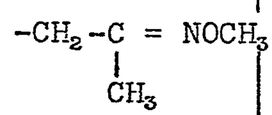 | |
| 250 | 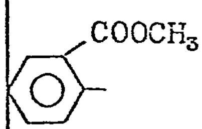 | 0 | H | H | $OCH_3$ | $-CH_2-CO-CH_3$ | |
| 251 | 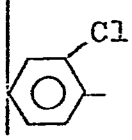 | 0 | H | H | $OCH_3$ | $-CH_2-CHO$ | |

| Bsp. Nr. | A | m | $R_1$ | $R_2$ | $R_3$ | $-B-R_4$ | Schmp. [°C] |
|---|---|---|---|---|---|---|---|
| 252 | 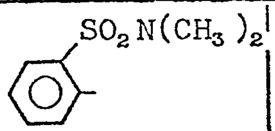 | O | H | H | $OCH_3$ | $-CH_2-CH_2-CHO$ | |
| 253 | $ClCH_2-CH_2-$ | O | H | H | $OCH_3$ | $-CH(OCH_3)_2$ | |
| 254 | $BrCH_2-CHBr-$ | O | H | H | $OCH_3$ | $-CH(OC_2H_5)_2$ | |
| 255 | $CH_3-CHCl-CH_2-$ | O | H | H | $OCH_3$ | 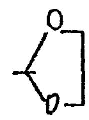 | |
| 256 | $CH_3-CH=CH-$ | O | H | H | $OCH_3$ | $-CH(OC_2H_5)_2$ | |
| 257 | $CH_3-CH=CH-$ | O | H | H | $OCH_3$ | $-CH_2-CO-CH_3$ | |
| 258 | $CH_3-CH=CH-$ | O | H | H | $OCH_3$ | $-CH_2-$ 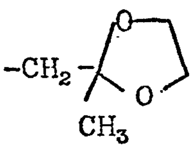 | |
| 259 | $CH_3-CHCl-CHCl$ | O | H | H | $OCH_3$ | $-CH(OC_2H_5)_2$ | |
| 260 | $CH_3-CHBr-CHBr-$ | O | H | H | $OCH_3$ | $-CH(OC_2H_5)_2$ | |
| 261 | $CH_3-CH=C(CH_3)-$ | O | H | H | $OC_2H_5$ | $-CH(OC_2H_5)_2$ | 64 - 66 |
| 262 | $CH_3-CHCl-CCl(CH_3)-$ | O | H | H | $OCH_3$ | 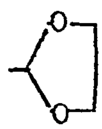 | |
| 263 | $CH_3CCl=C(CH_3)-$ | O | H | H | $OC_2H_5$ | $-CH_2-$ 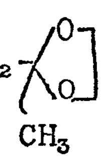 | |

| Bsp.-Nr. | A | m | $R_1$ | $R_2$ | $R_3$ | $-B-R_4$ | Schmp. /°C/ |
|---|---|---|---|---|---|---|---|
| 264 | $CH_3-CBr=C(CH_3)-$ | 0 | H | H | $OCH_3$ | 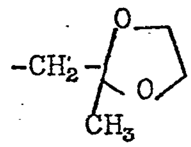 | |
| 265 | 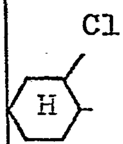 | 0 | H | H | $OCH_3$ | $-CH(OCH_3)_2$ | |
| 266 | 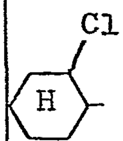 | 0 | H | H | $OCH_3$ | $-CH(SC_2H_5)_2$ | |
| 267 | 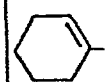 | 0 | H | H | $OCH_3$ | 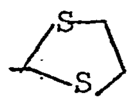 | |
| 268 | 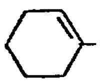 | 0 | H | H | $OCH_3$ | 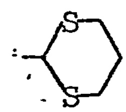 | |
| 269 | 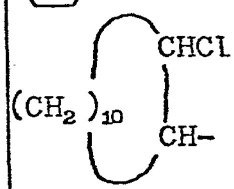 | 0 | H | H | $OCH_3$ | $-CH(OC_2H_5)_2$ | |
| 270 | 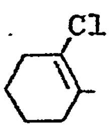 | 0 | H | H | $OCH_3$ | 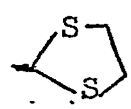 | |
| 271 | 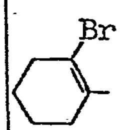 | 0 | H | H | $OCH_3$ | 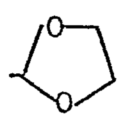 | |
| 272 | 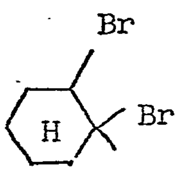 | 0 | H | H | $OCH_3$ | $-CH(OCH_3)_2$ | |
| 273 | $CH_3-CH=C(CH_3)-$ | 0 | H | H | $N(CH_3)_2$ | $-CH(OC_2H_5)_2$ | 83 - 86 |

| Bsp.-Nr. | A | m | $R_1$ | $R_2$ | $R_3$ | $-B-R_4$ | Schmp. [°C] |
|---|---|---|---|---|---|---|---|
| 274 | $CH_3-CHBr-CHBr$ | O | H | H | $OCH_3$ | $-CH_2-CO-CH_3$ | |
| 275 | $CH_3-CH=C(CH_3)-$ | O | H | H | $OCH_3$ | $-CH(OCH_3)_2$ | Oel |
| 276 | $CH_3-CHClCHCl-$ | O | H | H | $OCH_3$ | $-CH(OC_3H_7)_2$ | |
| 277 | $CH_3-CHClCHCl$ | O | H | H | $OCH_3$ | $-CH_2CHO$ | |
| 278 | 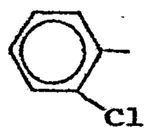 | O | H | $CH_3$ | $OCH_3$ | $-CH(OCH_3)_2$ | |
| 279 | " | O | H | $C_6H_5$ | $OCH_3$ | " | |
| 280 | 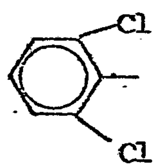 | O | H | H | $OCH_3$ | $-CHO$ | |
| 281 | 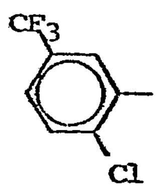 | O | H | H | $OCH_3$ | 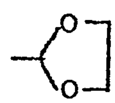 | |
| 282 | 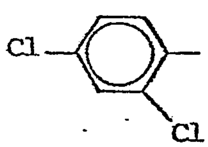 | O | H | H | $OCH_3$ | $-CH_2COCH_3$ | |
| 283 | 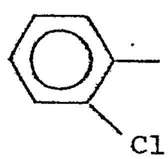 | O | H | H | $N(CH_3)_2$ | 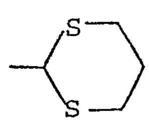 | 203 - 204 |
| 284 | 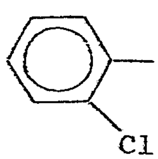 | O | H | H | $OCH_3$ | 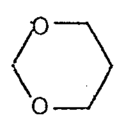 | 177 - 178 |
| 285 | 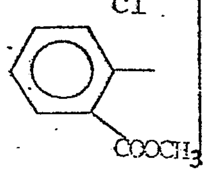 | O | H | H | $OCH_3$ | $CH_2-CH(OCH_3)_2$ | 127 - 128 |

Beispiel 286 (Ausgangsstoffe)

2-Amino-4-diethoxymethyl-6-methoxy-s-triazin

Zu einer Guanidino-O-methylisoharnstofflösung - hergestellt aus 15.25 g (0.1 mol) Guanidino-O-methylisoharnstoffhydrochlorid, 80 ml absolutem Methanol und einer Lösung von 2.53 g (0.11 mol) Natrium in 40 ml Methanol - läßt man bei 0°C 17.6 g (0.1 mol) Diethoxyessigester zu tropfen. Man rührt 20 Stunden bei Raumtemperatur, filtriert, engt das Filtrat ein, nimmt den Rückstand mit 2 N Essigsäure auf, extrahiert mit Methylenchlorid, wäscht die organische Phase mit Wasser, trocknet mit Natriumsulfat, filtriert, engt ein und kristallisiert den Rückstand aus Ether/n-Hexan um. Man erhält 15.4 g (67.5 %) 2-Amino-4-diethoxymethyl-6-methoxy-s-triazin vom Schmelzpunkt 82 - 83°C.

In analoger Weise erhält man die folgenden Verbindungen der Formel IV, worin Z jeweils Stickstoff bedeutet:

| Bsp.-Nr. | $R_2$ | $R_3$ | B-$R_4$ | F.P. [°C] |
|---|---|---|---|---|
| 287 | H | $OCH_3$ | $-CH(OCH_3)_2$ | 120 - 122 |
| 288 | H | $OCH_3$ | 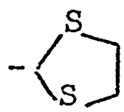 | 130 - 132 |
| 289 | H | $OCH_3$ | 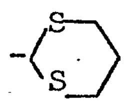 | 105 - 108 |
| 290 | H | $SCH_3$ | $-CH(OC_2H_5)_2$ | |
| 291 | H | Cl | $-CH(OC_2H_5)_2$ | |
| 292 | H | $CH_3$ | $-CH(OC_2H_5)_2$ | |
| 293 | H | $CH_3$ | 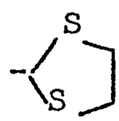 | |
| 294 | H | Cl | 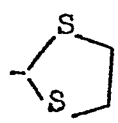 | |
| 295 | H | $OCH_3$ | 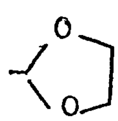 | 135 - 137 |
| 296 | H | $OCH_3$ | 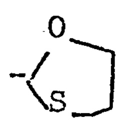 | 121 |

| Bsp.-Nr. | $R_2$ | $R_3$ | $-B-R_4$ | F.P. [°C] |
|---|---|---|---|---|
| 297 | H | $OCH_3$ | $-CH(SCH_3)_2$ | 75 - 79 |
| 298 | H | $OCH_3$ | $-C(OC_2H_5)_2$ with $CH_3$ | 110 -111 |
| 299 | H | $O(CH_2)_2OC_2H_5$ | $-CH(OCH_3)_2$ | 96 - 98 |
| 300 | H | $SCH_3$ | 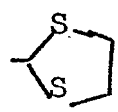 | |
| 301 | H | $OCH_3$ | $-CH{=}NOCH_3$ | |
| 302 | H | $OCH_3$ | $-CH{=}NNHC_6H_5$ | |
| 303 | H | $OCH_3$ | $-CH{=}NOH$ | |
| 304 | H | $O(CH_2)_2OC_2H_5$ | $-CH(OC_2H_5)_2$ | 77 |
| 305 | H | $OCH_3$ | $-CHO$ | |
| 306 | H | $N(CH_3)_2$ | $-CH(OC_2H_5)_2$ | 117-118 |
| 307 | H | $N(CH_3)_2$ | $-CH(OCH_3)_2$ | 176-177 |
| 308 | $CH_3$ | $OCH_3$ | $-CH(OC_2H_5)_2$ | |
| 309 | $CH_3$ | $OCH_3$ | 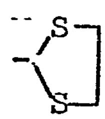 | |
| 310 | $C_2H_5$ | $OCH_3$ | 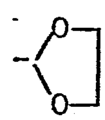 | |

| Bsp.-Nr. | $R_2$ | $R_3$ | $B-R_4$ | F.P. [°C] |
|---|---|---|---|---|
| 311 | $C_6H_5$ | $OCH_3$ | 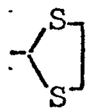 | |
| 312 | H | $OC_2H_5$ | $-CH(OC_2H_5)_2$ | 84 - 86 |
| 313 | H | $OC_2H_5$ | $-CH(OCH_3)_2$ | 102 - 103 |
| 314 | H | $OCH_3$ | 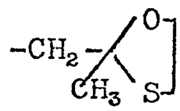 | 111 - 115 |
| 315 | H | $OCH_3$ | 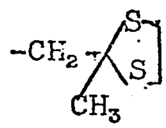 | 162 - 164 |
| 316 | H | $OCH_3$ | 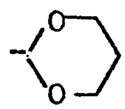 | 118 - 122 |
| 317 | H | $OCH_3$ | $-CH_2-CH(OC_2H_5)_2$ | 88 - 90 |
| 318 | H | $OCH_3$ | 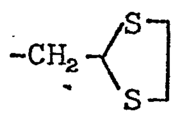 | 166 - 167 |
| 319 | H | $OCH_3$ | 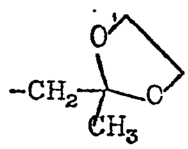 | 144 - 145 |
| 320 | H | $N(CH_3)_2$ | 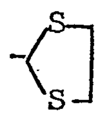 | 174 - 175 |
| 321 | H | $N(CH_3)_2$ | 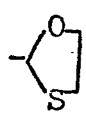 | 170 |

| Bsp.-Nr. | $R_2$ | $R_3$ | $B-R_4$ | F.P. [°C] |
|---|---|---|---|---|
| 322 | H | $N(CH_3)_2$ | $-CH(SCH_3)_2$ | 142 - 143 |
| 323 | H | $N(CH_3)_2$ | $-CH(SC_2H_5)_2$ | 126 - 128 |
| 324 | H | $OCH_3$ | 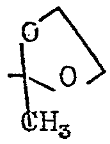 | 125 - 129 |
| 325 | H | $OCH_3$ | $-CH_2-CHO$ | |
| 326 | H | $OCH_3$ | $-CH_2CH(OCH_3)_2$ | |
| 327 | H | $OCH_3$ | $-CH_2-$ 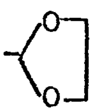 | |
| 328 | H | $OCH_3$ | $-CH_2CH_2CH(OCH_3)_2$ | 102 - 104 |
| 329 | H | $SCH_3$ | $-CH_2CH(OC_2H_5)_2$ | |
| 330 | H | Cl | $-CH_2CH(OC_2H_5)_2$ | |
| 331 | H | $N(CH_3)_2$ | - 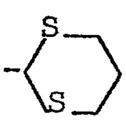 | 226 - 229 |

FORMULIERUNGSBEISPIELE

Beispiel A

Ein emulgierbares Konzentrat wird erhalten aus

15 Gew.-Teilen Wirkstoff
75 Gew.-Teilen Cyclohexan als Lösungsmittel
und 10 Gew.-Teilen oxethyliertes Nonylphenol (10 AeO) als Emulgator

Beispiel B

Ein in Wasser leicht dispergierbares benetzbares Pulver wird erhalten, indem man

25 Gew.-Teile Wirkstoff
64 Gew.-Teile Kaolinhaltiges Quarz als Inertstoff
und 1 Gew.-Teil oleoylmethyltaurinsaures Natrium als Netz und Dispergiermittel mischt und in einer Stiftmühle mahlt.

Beispiel C

Ein Stäubemittel wird erhalten, indem man

10 Gew.-Teile Wirkstoff
und 90 Gew.-Teile Talkum als Inertstoff mischt und in einer Schlagmühle zerkleinert.

BIOLOGISCHE BEISPIELE

1. Vorauflaufverfahren

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen

wurden in Form wäßriger Suspension oder Emulsionen auf die Erdoberfläche appliziert. Die Wasseraufwandmenge pro Topf entsprach dabei umgerechnet 600 - 800 l/ha. Nach der Behandlung wurden die Versuchstöpfe im Gewächshaus aufgestellt und die Versuchspflanzen unter guten Wachstumsbedingungen (Temperatur: 23 $\pm$ 1°C; rel. Luftfeuchte 60 - 80 %) kultiviert. Nach ca. 3 Wochen wurde die Pflanzenschädigung optisch bonitiert. Als Vergleich dienten dabei unbehandelte Kontrollen.

Die Schädigung der Unkrautpflanzen bzw. die Kulturpflanzenverträglichkeit wurde gemäß einem Schlüssel von 0 - 5 bonitiert.

Dabei bedeutet
0 = ohne Wirkung
1 = 0 - 20 % Wirkung
2 = 20 - 40 % Wirkung
3 = 40 - 60 % Wirkung
4 = 60 - 80 % Wirkung
5 = 80 - 100 % Wirkung

Tabelle 1

Vorauflaufwirkung der erfindungsgemäßen Verbindungen gegen mono- und dikotyle Unkräuter. Vergleiche zur unbehandelten Kontrolle

| Vbg gem. Bsp. | Dosis kg a.i./ha | herbizide Wirkung STM | AMR | LOM | ECG |
|---|---|---|---|---|---|
| 1 | 2,5 | 5 | 5 | 5 | 5 |
| 2 | " | 5 | 5 | 5 | 5 |
| 3 | " | 5 | 5 | 5 | 5 |
| 4 | " | 5 | 5 | 5 | 5 |
| 6 | " | 5 | 5 | 5 | 5 |
| 7 | " | 5 | 5 | 5 | 5 |
| 8 | " | 5 | 5 | 5 | 5 |
| 9 | " | 5 | 5 | 5 | 5 |
| 10 | " | 5 | 5 | 5 | 5 |
| 12 | " | 5 | 5 | 5 | 4 |
| 13 | " | 5 | 5 | 5 | 5 |
| 14 | " | 5 | 5 | 5 | 5 |
| 17 | " | 2 | 3 | 1 | 1 |
| 18 | " | 1 | 2 | 1 | 1 |
| 19 | " | 4 | 5 | 5 | 4 |
| 20 | " | 5 | 5 | 5 | 5 |
| 22 | " | 5 | 5 | 5 | 5 |
| 23 | " | 5 | 5 | 5 | 4 |
| 24 | " | 5 | 5 | 5 | 5 |
| 26 | " | 4 | 5 | 5 | 5 |
| 29 | " | 5 | 5 | 5 | 5 |
| 30 | " | 0 | 5 | 4 | 4 |
| 31 | " | 5 | 5 | 5 | 5 |
| 32 | " | 4 | 5 | 4 | 4 |
| 33 | " | 4 | 5 | 4 | 3 |
| 42 | " | 5 | 5 | 5 | 5 |
| 43 | " | 4 | - | 5 | - |
| 92 | " | 4 | 5 | 5 | 4 |
| 72 | " | 3 | 5 | 3 | 1 |
| 93 | " | 5 | 5 | 5 | 5 |

0098569

Fortsetzung Tabelle 1:

| Vbg gem. Bsp. | Dosis kg a.i./ha | herbizide Wirkung STM | AMR | LOM | ECG |
|---|---|---|---|---|---|
| 90 | 2,5 | 3 | 4 | 4 | 2 |
| 91 | " | 4 | 5 | 5 | 5 |
| 96 | " | 5 | 5 | 5 | 5 |
| 95 | " | 5 | 5 | 5 | 5 |
| 94 | " | 4 | 5 | 5 | 4 |
| 97 | " | 5 | 5 | 5 | 5 |
| 99 | " | 4 | 5 | 5 | 4 |
| 104 | " | 5 | 5 | 5 | 5 |
| 100 | " | 5 | 5 | 5 | 5 |
| 101 | " | 5 | 5 | 5 | 5 |
| 146 | " | 5 | 5 | 5 | 5 |
| 147 | " | 4 | - | 5 | - |
| 141 | " | 5 | 5 | 5 | 5 |
| 142 | " | 5 | 5 | 5 | 5 |
| 143 | " | 3 | 4 | 4 | 4 |
| 144 | " | 5 | 5 | 5 | 4 |
| 145 | " | 4 | 5 | 5 | 5 |
| 148 | " | 5 | 5 | 5 | 5 |
| 154 | " | 5 | 5 | 5 | 5 |
| 155 | " | 5 | 5 | 5 | 5 |
| 156 | " | 5 | 5 | 5 | 5 |
| 161 | " | 5 | 5 | 5 | 5 |
| 162 | " | 5 | 5 | 5 | 5 |
| 150 | " | 5 | 5 | 5 | 5 |
| 164 | " | 5 | 5 | 5 | 5 |
| 149 | " | 5 | 5 | 5 | 5 |
| 165 | " | 5 | 5 | 3 | 4 |
| 166 | " | 5 | 5 | 5 | 5 |
| 171 | " | 5 | 5 | 5 | 5 |
| 172 | " | 4 | 5 | 3 | 5 |
| 177 | " | 5 | 5 | 5 | 5 |
| 178 | " | 5 | 5 | 5 | 5 |
| 181 | " | 5 | 5 | 5 | 5 |

Fortsetzung Tabelle 1:

| Bsp. | Dosis kg a.i./ha | herbizide Wirkung STM | AMR | LOM | ECG |
|---|---|---|---|---|---|
| 182 | 2,5 | 5 | 5 | 5 | 5 |
| 186 | " | 5 | 5 | 5 | 5 |
| 187 | " | 5 | 5 | 5 | 5 |
| 188 | " | 4 | 2 | 3 | 3 |
| 193 | " | 5 | 5 | 5 | 5 |
| 194 | " | 5 | 5 | 4 | 4 |
| 195 | " | 5 | 5 | 4 | 3 |
| 199 | " | 5 | 5 | 5 | 5 |
| 200 | " | 5 | 5 | 5 | 5 |
| 201 | " | 5 | 5 | 5 | 5 |
| 204 | " | 5 | 5 | 5 | 5 |
| 205 | " | 2 | 1 | 1 | 1 |
| 211 | " | 3 | 4 | 3 | 3 |
| 212 | " | 2 | 2 | 2 | 3 |
| 213 | " | 5 | 5 | 5 | 5 |
| 214 | " | 5 | 5 | 5 | 5 |
| 215 | " | 5 | 5 | 5 | 5 |
| 218 | " | 5 | 5 | 5 | 5 |
| 219 | " | 5 | 5 | 5 | 5 |
| 220 | " | 5 | 5 | 5 | 5 |
| 237 | " | 5 | 5 | 4 | 5 |
| 238 | " | 5 | 5 | 5 | 5 |
| 239 | " | 5 | 5 | 4 | 4 |
| 240 | " | 5 | 5 | 4 | 5 |
| 221 | " | 5 | 5 | 5 | 5 |
| 228 | " | 5 | – | 3 | – |
| 230 | " | 2 | – | 2 | – |
| 229 | " | 4 | – | 0 | – |
| 192 | " | 5 | 5 | 5 | 5 |
| 283 | " | 1 | – | 0 | – |
| 246 | " | 5 | 5 | 5 | 5 |
| 245 | " | 5 | 5 | 5 | 5 |
| 284 | " | 5 | 5 | 5 | 5 |

Fortsetzung Tabelle 1:

| Bsp. | Dosis kg a.i./ha | herbizide Wirkung STM | AMR | LOM | ECG |
|---|---|---|---|---|---|
| 202 | 2,5 | 5 | 5 | 5 | 5 |
| 210 | " | 5 | 5 | 5 | 5 |
| 225 | " | 5 | 5 | 5 | 5 |
| 222 | " | 5 | - | 5 | 5 |

2. Nachauflaufverfahren

Samen von mono- und dikotylen Unkräutern wurden in Töpfen ausgesät und im Gewächshaus unter guten Wachstumsbedingungen angezogen. 3 Wochen nach der Aussaat wurden die Versuchspflanzen im Dreiblattstadium behandelt. Die als Spritzpulver bzw. als Emulsionskonzentrate formulierten erfindungsgemäßen Präparate wurden in verschiedenen Dosierungen auf die grünen Pflanzenteile gesprüht. Nach ca. 3 Wochen Standzeit im Gewächshaus unter optimalen Wachstumsbedingungen wurde die Wirkung der Präparate optisch im Vergleich zu unbehandelten Kontrollen bonitiert.

Tabelle 2

Nachauflaufwirkung der neuen erfindungsgemäßen Mittel gegen mono- und dikotyle Unkräuter. Vergleich zur unbehandelten Kontrolle.

| Bsp. | Dosierung | herbizide Wirkung | | | |
|---|---|---|---|---|---|
| | kg a.i./ha | STM | AMR | LOM | ECG |
| 1 | 2,5 | 5 | 5 | 5 | 5 |
| 14 | " | 4 | 5 | 4 | 4 |
| 18 | " | | | 2 | |
| 19 | " | 4 | 5 | 4 | 3 |
| 3 | " | 4 | 5 | 5 | 4 |
| 4 | " | 5 | 5 | 4 | 5 |
| 17 | " | 2 | 2 | 2 | |
| 2 | " | 5 | 5 | 5 | 5 |
| 6 | " | 5 | 5 | 5 | 5 |
| 8 | " | 4 | 5 | 5 | 3 |
| 9 | " | 5 | 5 | 5 | 5 |
| 20 | " | 5 | 5 | 5 | 5 |
| 74 | " | | | | |
| 72 | " | 2 | 3 | 2 | 1 |

Fortsetzung Tabelle 2:

Nachauflaufwirkung der neuen erfindungsgemäßen Mittel gegen mono- und dikotyle Unkräuter. Vergleich zur unbehandelten Kontrolle.

| Bsp. | Dosis kg a.i./ha | herbizide Wirkung STM | AMR | LOM | ECG |
|---|---|---|---|---|---|
| 22 | 2,5 | 5 | 5 | 5 | 5 |
| 31 | " | 3 | 4 | 5 | 4 |
| 32 | " | 2 | 4 | 5 | 2 |
| 23 | " | 2 | 4 | 5 | 2 |
| 12 | " | 5 | 5 | 5 | 5 |
| 26 | " | 3 | 5 | 5 | 2 |
| 30 | " | 5 | 5 | 5 | 3 |
| 29 | " | 0 | 5 | 2 | 2 |
| 24 | " | 5 | 5 | 5 | 5 |
| 10 | " | 5 | 5 | 5 | 5 |
| 93 | " | 3 | 5 | 5 | 5 |
| 91 | " | 3 | 5 | 5 | 5 |
| 96 | " | 3 | 5 | 5 | 4 |
| 95 | " | 3 | 5 | 5 | 5 |
| 94 | " | 2 | 5 | 3 | 4 |
| 42 | " | 4 | 5 | 5 | 5 |
| 97 | " | 3 | 5 | 4 | 5 |
| 141 | " | 3 | 5 | 3 | 5 |
| 142 | " | 4 | 5 | 5 | 5 |
| 92 | " | 4 | 5 | 4 | 4 |
| 33 | " | 3 | 5 | 4 | 4 |
| 143 | " | 3 | 5 | 4 | 3 |
| 144 | " | 5 | 5 | 5 | 4 |
| 90 | " | 4 | 5 | 4 | 3 |
| 145 | " | 5 | 5 | 5 | 5 |
| 104 | " | 5 | 5 | 5 | 5 |
| 7 | " | 5 | 5 | 5 | 5 |
| 100 | " | 5 | 5 | 5 | 5 |

Fortsetzung Tabelle 2:

| Bsp. | Dosis kg a.i./ha | herbizide Wirkung STM | ANR | LOM | ECG |
|---|---|---|---|---|---|
| 13 | 2,5 | 5 | 5 | 5 | 5 |
| 101 | " | 5 | 5 | 5 | 5 |
| 146 | " | 5 | 5 | 5 | 5 |
| 99 | " | 3 | 4 | 5 | 3 |
| 147 | " | 5 | 5 | 5 | 5 |
| 43 | " | 5 | 5 | 5 | 5 |
| 148 | " | 5 | 5 | 5 | 5 |
| 154 | " | 5 | 5 | 5 | 5 |
| 155 | " | 5 | 5 | 5 | 5 |
| 156 | " | 5 | 5 | 5 | 5 |
| 161 | " | 5 | 5 | 5 | 5 |
| 162 | " | 5 | 5 | 5 | 5 |
| 150 | " | 3 | 5 | 4 | 5 |
| 164 | " | 5 | 5 | 5 | 5 |
| 149 | " | 5 | 5 | 4 | 4 |
| 165 | " | 5 | 5 | 3 | 5 |
| 166 | " | 5 | 5 | 5 | 5 |
| 171 | " | 5 | 5 | 5 | 5 |
| 172 | " | 4 | 5 | 3 | 5 |
| 177 | " | 5 | 5 | 5 | 5 |
| 178 | " | 5 | 5 | 5 | 5 |
| 181 | " | 5 | 5 | 5 | 5 |
| 182 | " | 5 | 5 | 5 | 5 |
| 186 | " | 5 | 5 | 5 | 5 |
| 187 | " | 4 | 5 | 5 | 5 |
| 188 | " | 0 | 2 | 2 | 2 |
| 193 | " | 5 | 5 | 5 | 5 |
| 194 | " | 4 | 5 | 4 | 4 |
| 195 | " | 3 | 3 | 2 | 2 |
| 199 | " | 5 | 5 | 4 | 4 |
| 200 | " | 5 | 5 | 5 | 5 |
| 201 | " | 5 | 5 | 5 | 5 |

Fortsetzung Tabelle 2:

| Bsp. | Dosis kg a.i./ha | herbizide Wirkung STM | AMR | LOM | ECG |
|---|---|---|---|---|---|
| 204 | 2,5 | 5 | 5 | 5 | 5 |
| 205 | " | 3 | 1 | 2 | 1 |
| 211 | " | 3 | 3 | 2 | 1 |
| 212 | " | 3 | 2 | 2 | 2 |
| 213 | " | 5 | 3 | 4 | 3 |
| 214 | " | 5 | 3 | 4 | 3 |
| 215 | " | 5 | 1 | 5 | 4 |
| 218 | " | 5 | 4 | 5 | 5 |
| 219 | " | 5 | 5 | 5 | 5 |
| 220 | " | 5 | 4 | 4 | 3 |
| 237 | " | 5 | 4 | 4 | 4 |
| 238 | " | 5 | 5 | 5 | 5 |
| 239 | " | 5 | 5 | 3 | 4 |
| 240 | " | 4 | 4 | 3 | 2 |
| 221 | " | 5 | 5 | 5 | 5 |
| 228 | " | 5 | 3 | 3 | 2 |
| 230 | " | 2 | 1 | 1 | 0 |
| 229 | " | 2 | 1 | 2 | 0 |
| 192 | " | 5 | 5 | 5 | 4 |
| 283 | " | 0 | 0 | 0 | 0 |
| 246 | " | 5 | 5 | 5 | 5 |
| 245 | " | 5 | 5 | 5 | 4 |
| 284 | " | 5 | 5 | 5 | 5 |
| 202 | " | 5 | 5 | 5 | 5 |
| 210 | " | 5 | 5 | 5 | 5 |
| 225 | " | 5 | 5 | 5 | 5 |
| 222 | " | 5 | - | 5 | 5 |

Abkürzungen:

STM = Stellaria media
AMR = Amaranthus retroflexus
LOM = Lolium multiflorum
ECC = Echinochloa crus galli

PATENTANSPRÜCHE:

1. Verbindungen der Formel

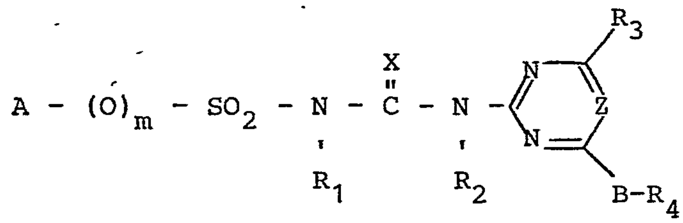

(I)

worin

A einen (ggfs. durch bis zu 6 Halogenatomen substituierten) gesättigten oder ungesättigten aliphatischen Rest mit bis zu 10 C-Atomen, der auch durch Sauerstoff unterbrochen sein kann; einen gesättigten cycloaliphatischen Rest mit 3 bis 12 C-Atomen oder einen ein- oder mehrfach ungesättigten Rest mit 5 - 12 C-Atomen, die gegebenenfalls durch bis zu 4 Halogenatome und/oder durch einen oder mehrere $(C_1-C_4)$-Alkyl- oder Halogenalkyl (letztere mit 1 - 3 Halogenatomen) bzw. durch einen $(C_1-C_4)$-Alkoxycarbonylrest substituiert sein können; einen bicyclischen gesättigten oder ein- oder zweifach ungesättigten aliphatischen Rest mit 7 bis 12 C-Atomen, welcher gegebenenfalls bis zu 6 Halogenatome oder eine oder mehrere $(C_1-C_4)$-Alkylreste tragen kann oder in dem eine $CH_2$-Brücke durch Sauerstoff ersetzt sein kann; oder einen Rest der Formel

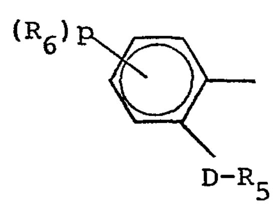

m null oder 1,

X Sauerstoff oder Schwefel,

$R_1$ Wasserstoff oder $(C_1-C_4)$-Alkyl,
$R_2$ Wasserstoff, $(C_1-C_4)$-Alkyl oder Phenyl, das durch Halogen, $(C_1-C_4)$-Alkyl, $(C_1-C_4)$-Alkoxy, $NO_2$, $(C_1-C_4)$-Alkylthio und/oder Di-$(C_1-C_4)$-alkylamino substituiert sein kann,
$R_3$ Wasserstoff, Halogen, $NO_2$, CN, $(C_1-C_4)$-Alkylamino, Di-$(C_1-C_4)$-alkyl-amino, $(C_1-C_4)$-Alkyl, das ggfs. durch Halogen, $(C_1-C_3)$-Alkoxy, $(C_1-C_3)$-Alkylthio, $(C_1-C_3)$-Alkylamino, Di-$(C_1-C_3)$-alkylamino oder $(C_1-C_4)$-Alkoxycarbonyl substituiert sein kann; $(C_1-C_4)$-Alkoxy oder $(C_1-C_4)$-Alkylthio, die ggfs. durch Halogen, $(C_1-C_4)$-Alkoxy oder $(C_1-C_4)$-Alkoxycarbonyl substituiert sein können, Allyloxy oder $(C_1-C_4)$-Alkoxycarbonyl;
B und D unabhängig voneinander jeweils eine einfache chemische Bindung oder eine Methylen- oder Ethylengruppe, die durch Halogen, $(C_1-C_4)$-Alkyl, $(C_1-C_4)$-Alkoxy, $(C_1-C_4)$-Alkylthio, $(C_1-C_4)$-Alkylamino oder Di-$(C_1-C_4)$-alkylamino substituiert sein kann;
$R_4$ ein Rest der Formel $-CO-R_7$;
$R_5$ Halogen, $NO_2$, $(C_1-C_4)$-Alkyl, $(C_1-C_4)$ Halogenalkyl oder -alkoxy mit jeweils bis zu 5 Halogenatomen, $(C_1-C_4)$-Alkoxy, $(C_2-C_5)$-Alkenoxy, $(C_1-C_3)$-Alkylthio, -Alkylsulfinyl oder -Alkylsulfonyl, $-SO_2N[(C_1-C_4)\text{-Alkyl}]_2$ oder $(C_1-C_4)$-Alkoxycarbonyl, einen Phenyl- bzw. Phenoxy-, Diphenyl- bzw. Diphenoxy-, Phenoxyphenyl- bzw. Phenoxyphenoxyrest, worin die Phenylreste durch Halogen, $NO_2$, $(C_1-C_4)$-Alkyl oder Alkoxy substituiert sein können,
$R_6$ gleich oder verschiedene Reste der Gruppe Halogen, $CF_3$, $NO_2$, $(C_1-C_4)$-Alkyl, $(C_1-C_4)$-Alkoxy oder $(C_2-C_5)$-Alkenoxy;
p 0 - 3;
$R_7$ Wasserstoff, $(C_1-C_4)$-Alkyl, $(C_1-C_4)$-Halogenalkyl, Phenyl, Halogenphenyl, $(C_1-C_4)$-Alkylphenyl oder $(C_1-C_4)$-Alkoxyphenyl,
Z Stickstoff oder eine Methin-Gruppe bedeuten sowie die

funktionellen Derivate dieser Verbindungen und ihre physiologisch verträglichen Salze mit Basen und Säuren.

2. Verfahren zur Herstellung von Verbindungen der Formel I, dadurch gekennzeichnet, daß man

a) Verbindungen der Formel

$$A - (O)_m - SO_2 - N = C = O \qquad (II),$$

oder

$$A - (O)_m - SO_2 - \underset{R_1}{\underset{|}{N}} - \overset{X}{\overset{\|}{C}} - Y \qquad (III)$$

wobei für X = S Y = Cl und für X = O Y = Cl, $O(C_1-C_4)$-Alkyl oder $OC_6H_5$ bedeutet mit Verbindungen der Formel

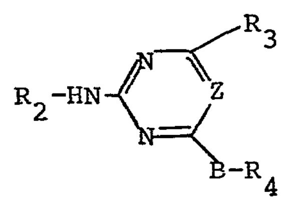

(IV)

oder

b) Verbindungen der Formel

$$A - (O)_m - SO_2 - \underset{R_1}{\underset{|}{NH}} \qquad (V)$$

mit Verbindungen der Formel

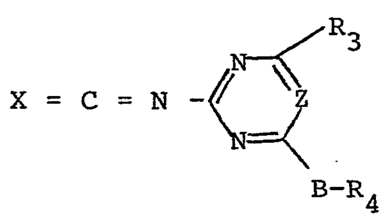

(VI)

oder

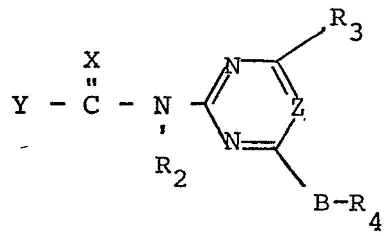

(VII)

umsetzt, wobei in Formel VII $R_2$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_1$-Position oder Salzbildung in andere Verbindungen der Formel I überführt, oder

c) Verbindungen der Formel

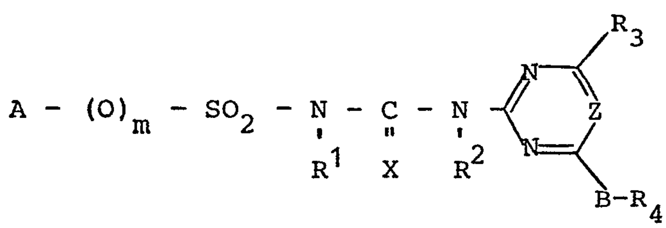

(I)

durch Umsetzung mit Alkoholen, Glycolen, Mercaptanen, Aminen, Hydroxylaminen, Anilinen, Hydrazinen oder Semicarbaziden in andere funktionelle Derivate der Formel I überführt.

3. Herbizide und wachstumsregulierende Mittel, gekennzeichnet durch einen Gehalt an einer Verbindung der Formel I.

4. Verwendung von Verbindungen der Formel I zur Bekämpfung von unerwünschtem Pflanzenwuchs bzw. zur Wachstumsregulierung von Nutzpflanzen.

5. Verfahren zur Bekämpfung von unerwünschtem Pflanzenwuchs bzw. zur Wachstumsregulierung von Nutzpflanzen, dadurch gekennzeichnet, daß man auf die zu behandelnden Pflanzen oder die Anbaufläche eine wirksame Menge einer Verbindung der Formel I aufbringt.

6. Verbindungen der Formel IV, worin $R_2$, $R_3$, $R_4$ und B die angegebenen Bedeutungen haben und Z = Stickstoff bedeutet.

PATENTANSPRÜCHE Österreich:

1. Verfahren zur Herstellung von Verbindungen der Formel I

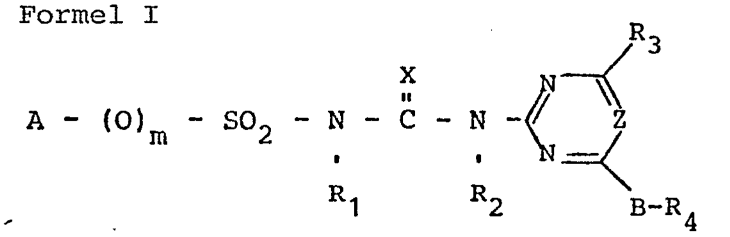

(I)

worin

A einen (ggfs. durch bis zu 6 Halogenatomen substituierten) gesättigten oder ungesättigten aliphatischen Rest mit bis zu 10 C-Atomen, der auch durch Sauerstoff unterbrochen sein kann; einen gesättigten cycloaliphatischen Rest mit 3 bis 12 C-Atomen oder einen ein- oder mehrfach ungesättigten Rest mit 5 - 12 C-Atomen, die gegebenenfalls durch bis zu 4 Halogenatome und/oder durch einen oder mehrere $(C_1-C_4)$-Alkyl- oder Halogenalkyl (letztere mit 1 - 3 Halogenatomen) bzw. durch einen $(C_1-C_4)$-Alkoxycarbonylrest substituiert sein können; einen bicyclischen gesättigten oder ein- oder zweifach ungesättigten aliphatischen Rest mit 7 bis 12 C-Atomen, welcher gegebenenfalls bis zu 6 Halogenatome oder eine oder mehrere $(C_1-C_4)$-Alkylreste tragen kann oder in dem eine $CH_2$-Brücke durch Sauerstoff ersetzt sein kann; oder einen Rest der Formel

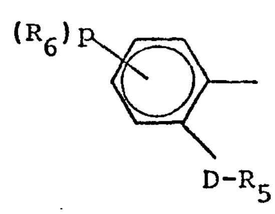

m null oder 1,

X Sauerstoff oder Schwefel,

$R_1$ Wasserstoff oder $(C_1-C_4)$-Alkyl,

$R_2$ Wasserstoff, $(C_1-C_4)$-Alkyl oder Phenyl, das durch Halogen, $(C_1-C_4)$-Alkyl, $(C_1-C_4)$-Alkoxy, $NO_2$, $(C_1-C_4)$-Alkylthio und/oder Di-$(C_1-C_4)$-alkylamino substituiert sein kann,

$R_3$ Wasserstoff, Halogen, $NO_2$, CN, $(C_1-C_4)$-Alkylamino, Di-$(C_1-C_4)$-alkyl-amino, $(C_1-C_4)$-Alkyl, das ggfs. durch Halogen, $(C_1-C_3)$-Alkoxy, $(C_1-C_3)$-Alkylthio, $(C_1-C_3)$-Alkylamino, Di-$(C_1-C_3)$-alkylamino oder $(C_1-C_4)$-Alkoxycarbonyl substituiert sein kann; $(C_1-C_4)$-Alkoxy oder $(C_1-C_4)$-Alkylthio, die ggfs. durch Halogen, $(C_1-C_4)$-Alkoxy oder $(C_1-C_4)$-Alkoxycarbonyl substituiert sein können, Allyloxy oder $(C_1-C_4)$-Alkoxycarbonyl;

B und D unabhängig voneinander jeweils eine einfache chemische Bindung oder eine Methylen- oder Ethylengruppe, die durch Halogen, $(C_1-C_4)$-Alkyl, $(C_1-C_4)$-Alkoxy, $(C_1-C_4)$-Alkylthio, $(C_1-C_4)$-Alkylamino oder Di-$(C_1-C_4)$-alkylamino substituiert sein kann;

$R_4$ ein Rest der Formel $-CO-R_7$;

$R_5$ Halogen, $NO_2$, $(C_1-C_4)$-Alkyl, $(C_1-C_4)$ Halogenalkyl oder -alkoxy mit jeweils bis zu 5 Halogenatomen, $(C_1-C_4)$-Alkoxy, $(C_2-C_5)$-Alkenoxy, $(C_1-C_3)$-Alkylthio, -Alkylsulfinyl oder -Alkylsulfonyl, $-SO_2N[(C_1-C_4)\text{-Alkyl}]_2$ oder $(C_1-C_4)$-Alkoxycarbonyl, einen Phenyl- bzw. Phenoxy-, Diphenyl- bzw. Diphenoxy-, Phenoxyphenyl- bzw. Phenoxyphenoxyrest, worin die Phenylreste durch Halogen, $NO_2$, $(C_1-C_4)$-Alkyl oder Alkoxy substituiert sein können,

$R_6$ gleich oder verschiedene Reste der Gruppe Halogen, $CF_3$, $NO_2$, $(C_1-C_4)$-Alkyl, $(C_1-C_4)$-Alkoxy oder $(C_2-C_5)$-Alkenoxy;

p 0 - 3;

$R_7$ Wasserstoff, $(C_1-C_4)$-Alkyl, $(C_1-C_4)$-Halogenalkyl, Phenyl, Halogenphenyl, $(C_1-C_4)$-Alkylphenyl oder $(C_1-C_4)$-Alkoxyphenyl,

Z Stickstoff oder eine Methin-Gruppe bedeuten sowie die

funktionellen Derivate dieser Verbindungen und ihrer physiologisch verträglichen Salze mit Basen und Säuren,

dadurch gekennzeichnet, daß man

a) Verbindungen der Formel

$A - (O)_m - SO_2 - N = C = O$ (II),

oder

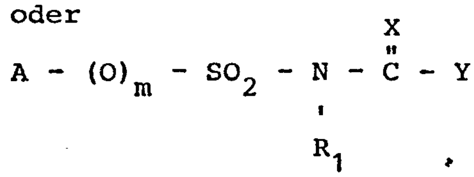 (III)

wobei für X = S Y = Cl und für X = O Y = Cl, $O(C_1-C_4)$-Alkyl oder $OC_6H_5$ bedeutet mit Verbindungen der Formel

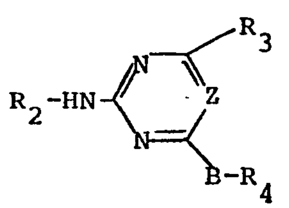 (IV)

oder

b) Verbindungen der Formel

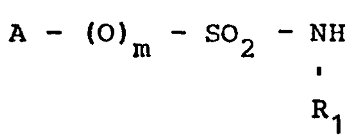 (V)

mit Verbindungen der Formel

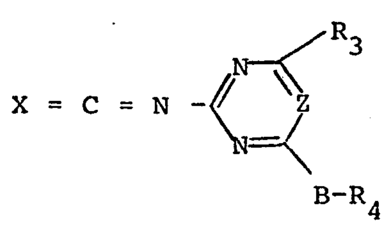 (VI)

oder

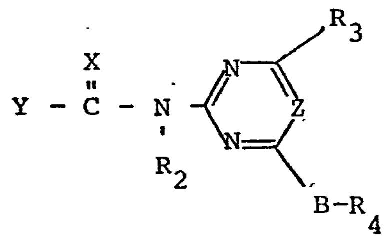

(VII)

umsetzt, wobei in Formel VII $R_2$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_1$-Position oder Salzbildung in andere Verbindungen der Formel I überführt, oder

c) Verbindungen der Formel

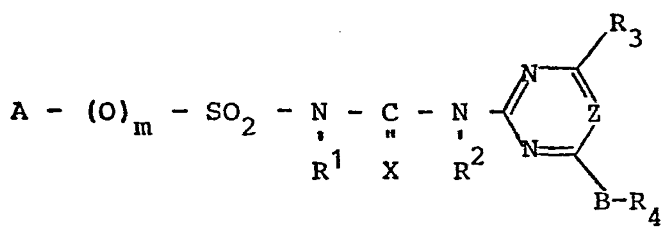

(I)

durch Umsetzung mit Alkoholen, Glycolen, Mercaptanen, Aminen, Hydroxylaminen, Anilinen, Hydrazinen oder Semicarbaziden in andere funktionelle Derivate der Formel I überführt.

2. Herbizide und wachstumsregulierende Mittel, gekennzeichnet durch einen Gehalt an einer Verbindung der Formel I von Anspruch 1.

3. Verwendung von Verbindungen der Formel I von Anspruch 1 zur Bekämpfung von unerwünschtem Pflanzenwuchs bzw. zur Wachstumsregulierung von Nutzpflanzen.

4. Verfahren zur Bekämpfung von unerwünschtem Pflanzenwuchs bzw. zur Wachstumsregulierung von Nutzpflanzen, dadurch gekennzeichnet, daß man auf die zu behandelnden Pflanzen oder die Anbaufläche eine wirksame Menge einer Verbindung der Formel I aufbringt.